(19) Europäisches Patentamt / European Patent Office / Office européen des brevets

(11) **EP 4 233 765 A1**

(12) **EUROPEAN PATENT APPLICATION**

(43) Date of publication:
**30.08.2023 Bulletin 2023/35**

(21) Application number: **22158571.4**

(22) Date of filing: **24.02.2022**

(51) International Patent Classification (IPC):
**A61B 34/30** (2016.01)     **F03G 7/06** (2006.01)

(52) Cooperative Patent Classification (CPC):
**A61B 34/30; F03G 7/004;** A61B 34/73;
A61B 2017/00345; A61B 2017/00411;
A61B 2017/00871; A61B 2017/00876;
A61B 2034/303

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Max-Planck-Gesellschaft zur Förderung der Wissenschaften e.V.**
**80539 München (DE)**

(72) Inventors:
• **Sitti, Metin**
  **Stuttgart (DE)**
• **Hu, Wenqi**
  **Stuttgart (DE)**
• **Li, Mington**
  **Stuttgart (DE)**
• **Tang, Yichao**
  **Shanghai (CN)**

(74) Representative: **Manitz Finsterwald Patent- und Rechtsanwaltspartnerschaft mbB Martin-Greif-Strasse 1 80336 München (DE)**

(54) **COILED DEVICE**

(57)   The invention relates to a device composed of one or more structures, each of said structures comprising at least three layers a core, an active sheath; and a protective sheath, wherein the core is formed by nylon fibers, wherein the active sheath comprises a resin, with graphene oxide (GO) and a first type of magnetic particles being respectively embedded in the resin; and wherein the protective sheath is formed from an elastomeric material that protects the inner layers from an external environment.

EP 4 233 765 A1

**Description**

The invention relates to a device composed of one or more structures. The invention further relates to a method of forming a device having one or more structures.

Wireless small-scale and soft-bodied medical devices that are as small as a few millimetres or even smaller can already safely and adaptively be navigated through confined spaces, such as inside machines with canals and channels comprising a small diameter or even inside a human or animal body. This unique capability can also potentially be advantageous in diverse medical applications such as minimally invasive surgery and local on-demand therapeutic operations.

Various optical, thermal and also acoustic soft actuation methods have already been explored for such miniature devices. While the softness of their body endows these devices with the capability to have large programmed deformations and safe interaction with the environment, it also limits their output force and weight-normalized work capacity.

Typically, soft devices exhibit small force output and large deformation. The material softness makes it hard to store and release large amounts of mechanical energy. Hence, they cannot be used in device and robot applications requiring large force output and high work capacity.

According to one example, the output force of existing magnetic soft devices was calculated to saturate around 60 $\mu$N, irrespective of increasing the external magnetic field amplitude.

However, some medical procedures, such as pinching, clamping and cutting require devices with much higher force output, i.e. a force output that is larger than 1 N, which is about $10^5$ times higher than the maximum capacity of the previously reported magnetic soft devices.

At scales that are in the range of a few centimeter and larger, coiled artificial muscles have proven to be promising devices that can be produced by continuously twisting polymer fibers into a coiled shape. Recent works have proposed that the coiled muscles can have higher work capacities and larger output forces than previously proposed approaches. Specifically, the coiled muscles showed a work capacity that was up to 50 times larger than the work capacity of biological skeletal muscles. Furthermore, the coiled muscles could deliver an output that was more than 1000 times higher than their own weight when said muscles were composed of carbon nanotubes, shape memory polymers and fishing lines.

Additionally, the output force and work capacity of said coiled muscles could be greatly enhanced by tailoring the component or structure of the precursor fiber of the coiled muscle, e.g. by adding graphene oxide (GO) platelets to the precursor fiber or by designing a tough sheath on its surface. Such coiled muscles are poised to advance the fields of humanoid robots, prosthetic limbs and microfluidic devices.

In view of this background, it is an object of the invention to provide devices with a large output force and a high work capacity.

This object is solved by the subject matter of the independent claims.

In particular, the device according to the invention is composed of one or more structures with each of said structures comprising at least three layers. That is, the device comprises at least a core, an active sheath, and a protective sheath. The core is formed by at least one nylon fiber, and the active sheath comprises a resin, with graphene oxide (GO) and a first type of magnetic particles being respectively embedded in the resin. Furthermore, the protective sheath is formed from an elastomeric material that protects the inner layers from an external environment.

Thus, in other words, the device according to the invention comprises one or more structures, e.g. artificial coiled muscles, that is/are integrated into the device, e.g. a miniature soft device, such that the applications of the device can be extended to the ones that require a large output force and a high work capacity.

The one or more structures are built at least as a tri-layer structure comprising a core, an active sheath and a protective sheath. Each layer has a specific function.

By way of example, the function of the core is to provide the inherent function of the device, i.e. if a clamping or gripping function of the device is desired then the core can be formed by a structure enabling such a clamping or gripping function, e.g. in the form of a coiled structure.

The active sheath is to permit an external actuation of the device either in a tethered, or more preferably in an untethered manner, e.g. via the application of magnetic and/or electromagnetic fields.

Finally, the function of the protective sheath is to protect the core and the active sheath from external influences, such as the fluids present in a human body.

The core is formed by at least one nylon fiber. Such fibers have proven to be advantageous because it can, for example, be twisted in order to be utilized as a coiled structure such as the ones described above.

The active sheath comprises a resin with graphene oxide (GO) and a first type of magnetic particles, in particular nanoparticles, being respectively embedded therein. The first type of magnetic particles can also comprise paramagnetic and/or superparamagnetic (nano)particles. The combination of GO particles and magnetic particles can increase the toughness of the active sheath and can enable e.g. wireless heating, such as RF-magnetic heating, respectively. The heating process is necessary to activate the structure.

Furthermore, the introduction of ((super)para)magnetic particles and GO into the active layer can contribute to an untethered actuation with a large force output up to more than 3 N as well as a high work capacity which can reach values of up to 3.5 kJ/kg.

The function of the protective sheath is to protect the inner layers from external environmental influences. Fluid environments, for example, can reduce the heating efficiency of the device, thereby affecting the actuation strain and output force of the device. The protective layer can be configured to protect the inner layers from such an effect. Another function of the protective layer can be to protect the inner layers from structural damages that could possibly occur when the device is in motion, for example, because of mechanical collisions with other structures. Another function of the protective layer can also be to protect the external environment from the heat generated by the active sheath. It could, for example, be shown that the surface temperature of the device without the protective layer rises up to above 120°C while the surface temperature of the device including the protective layer could be held at about 60°C or even less, such as e.g. 30°C, when the temperature was measured at a small distance, e.g. 2 mm, from the surface of the device.

Thus, it can be concluded that the protective layer of the structure comprises several important functions.

In this connection it should also be noted that by integrating one or more of these structures into different devices a great variety of miniature devices can be realized such as for example suture devices, scissors, drillers and energy storing bistable structures which all require a high force output as well as work capacity.

According to a first embodiment of the invention the device further comprises a fourth layer with the fourth layer being a magnetization sheath. Said fourth layer can be used, for example, to encode a magnetization profile on the device to enable magnetic deformation and high output contractile force.

According to another embodiment the magnetization sheath comprises a combination of magnetic particles together with an elastomer, such as a combination of NdFeB and PDMS. Other examples for the magnetic particles of the fourth layer can be permanent magnetic particles such as AlNiCo magnets and/or SmCo magnets. The elastomer matrix can also include silicone rubber, such as Ecoflex 00-30™, Ecoflex 00-50™ or Dragon skin 30™.

It may further be possible that the resin of the active sheath comprises a urethane casting resin, such as Crystal Clear™, or nylon. The matrix materials of the active sheath, i.e. the resin of the active sheath, must comprise tough materials that are very durable.

According to another embodiment the first type of magnetic particles is formed by $Fe_3O_4$. That is, the magnetic particles present in the active sheath are formed by $Fe_3O_4$ particles which can be used to generate heat under, for example, RF-magnetic heating. Such a heating process can, for example, cause a coiled structure to contract such that a movement can be generated.

The first type of magnetic particles, i.e. of the $Fe_3O_4$ particles, can comprise a size selected in the range of 10 to 500 nm, especially in the range of 20 to 200 nm, in particular in the range of 50 to 100 nm.

According to another embodiment the device comprises a second type of magnetic particles being formed by NdFeB, with said second type of magnetic particles comprising a size selected in the range of 0,1 to 10 μm, especially in range of 2 to 6 μm. The second type of magnetic particles can be embedded in the fourth layer if present. Such particles can be used to, for example, propel different devices according to the invention under magnetic torque and gradient. The average diameter of such a particle usually lies around 5 μm.

It may further be possible that the active sheath comprises a thickness in the range of 50 to 300 μm, in particular of 100 to 250 μm, especially 200 μm.

The active sheath can comprise a GO concentration selected in the range of 1 to 6 wt%, especially in the range of 2 to 4 wt%. Increasing the GO concentration of the device can enhance the output performance of the device. However, as the GO concentration increases the active sheath tends to become more fragile and brittle. Hence, the correct choice of GO concentration is not trivial.

In a further embodiment the active sheath comprises a magnetic particle concentration of the first type of magnetic particles selected in the range of 10 to 30 wt%. The heating efficiency of the active sheath increases with increasing concentration of the first type of magnetic particles, which is advantageous for the device according to the invention. However, if said concentration is too high, the device tends break more easily. Therefore, the aforementioned range of 10 to 30 wt% has proven to be preferable.

The device can also comprise a coiled shape. As already mentioned in the introductory part of this application coiled structures have proven to have a higher work capacity and larger output forces.

According to another embodiment the nylon fibers comprise a diameter selected in the range of 0.1 to 0.5 mm, especially of 0.2 to 0.5 mm, in particular of 0.45 mm.

In this connection it is further noted that the nylon fibers can have a length selected in the range of 1 to 100 cm, in particular in the range of 1 to 50 cm, especially in the range of 5 to 40 cm.

According to a further embodiment the device comprises a diameter selected in the range of 0.1 to 10 mm, in particular of 0.4 to 5 mm, especially of 0.4 to 1.2 mm.

In this connection it should be noted that the nylon fiber as described in the foregoing can be twisted a plurality of times to form the coiled structure, by way of example the number of twists can be selected in the range of 100 to 10000 twists, i.e. the coiled structure comprises between 100 to 10000 turns of a single nylon fiber in each core. The exact number of turns depends on the length of the fiber that is supposed to be twisted. For a fiber length in the range of, for example, 1 to 50 cm about 100 to 1000 turns are needed.

According to yet another embodiment the device comprises at least one functional component such as a frame, blades, bars, a screw, a chassis and/or combinations of the foregoing. Such functional components can enable the device to be utilized in a variety of applications such as untethered miniature soft (medical) devices and robot applications, thereby expanding the field of applications of currently known devices. That is, different examples of devices that can be realized are sutures, drillers, scissors, clampers and even multi-linked devices, with each device having a corresponding potential function.

According to a further aspect of the invention a method of forming a device having one or more three layered structures is provided. the method comprises the steps of providing a fiber; coating the fiber with an active sheath material, wherein the active sheath material comprises a resin, with graphene oxide (GO) and a first type of magnetic particles being embedded in the resin; further coating the fiber with a protective sheath material, wherein the protective sheath material comprises elastomeric material; and continuously twisting and coiling said coated fiber to form the device. Optionally it is also possible to cure said coated fiber before twisting and twirling it.

According to one embodiment of the invention the method further comprises the step of coating the fiber with an additional magnetic sheath before coiling the coated fiber. Said additional magnetic sheath a combination of magnetic particles together with an elastomer, such as a combination of NdFeB and PDMS. Other examples for the magnetic particles of the fourth layer can be permanent magnetic particles such as AlNiCo magnets and/or SmCo magnets. The elastomer matrix can also include silicone rubber, such as Ecoflex 00-30™, Ecoflex 00-50™ or Dragon skin 30™.

It is further possible that the step of coiling said coated fibers comprises at least 250 turns, preferably 300 to 400 turns. The exact number of turns usually depends on the length of the fiber. For example, 100 to 800 turns are needed to coil a fiber of length between 5 to 40 cm.

Further embodiments of the invention are described in the following description of the Figures. The invention will be explained in the following in detail by means of embodiments and with reference to the drawing in which is shown:

| | |
|---|---|
| Fig. 1: | The design, fabrication process, actuation modes and actuation mechanism of a magnetically heated coiled muscle structure; |
| Fig. 2: | a characterization of the coiled muscle structure performance; |
| Fig. 3: | a wireless suturing device; |
| Fig. 4: | a wireless medical scissor device; |
| Fig. 5: | a wireless medical driller device; |
| Fig. 6: | a wireless bistable clamper; |
| Fig. 7: | a multi-linked coiled muscle structure; |
| Fig. 8: | a comparison of different types of soft and muscle devices in terms of actuation strain, work capacity and energy density; |
| Fig. 9: | the modeling of a magnetic soft actuator device; |
| Fig. 10: | a calculation of a magnetic soft actuator device work capacity; |
| Fig. 11: | the thermal characterization of a coiled muscle structure; |
| Fig. 12: | a SEM morphology characterization; |
| Fig. 13: | an output force characterization; |
| Fig. 14: | the tensile strain and torsional stroke of the coiled muscle structure; |
| Fig. 15: | a reversible torsional actuation; |
| Fig. 16: | the strain distribution of the coiled muscle structure in a cross-sectional view; |
| Fig. 17: | a characterization of the coiled muscle structure performance in deionized water; |
| Fig. 18: | a performance evaluation and application prospects of the coiled muscle structure; |
| Fig. 19: | force tests for a suturing device; |
| Fig. 20: | design of a reversible clamper device; |
| Fig. 21: | a precursor fiber design of the multi-linked coiled muscle structure; |
| Fig. 22: | a surface walking locomotion of the multi-linked coiled muscle structure; |
| Fig. 23: | a characterization of the active sheath thickness; |
| Fig. 24: | the schematic of a measurement; |
| Fig. 25: | different methods for measuring the actuation strain; |
| Fig. 26: | a calculation of the output force of the magnetic soft actuator device; |
| Fig. 27: | a calculation of the actuation strain and work capacity of the coiled muscle structure; |
| Fig. 28: | a comparison of theoretical and experimental results for the coiled muscle structure; |
| Fig. 29: | measurement schematic for the clamper device; |
| Fig. 30: | ways for optimization of the bistable design; |

Fig. 31:    the static clamping force and potential energy of the bistable clamper device; and

Fig. 32:    a characterization of a pre-stretched elastomer.

In the following the same reference numerals will be used for parts having the same or equivalent function. Any statements made having regard to the direction of a component are made relative to the position shown in the drawing and can naturally vary in the actual position of use.

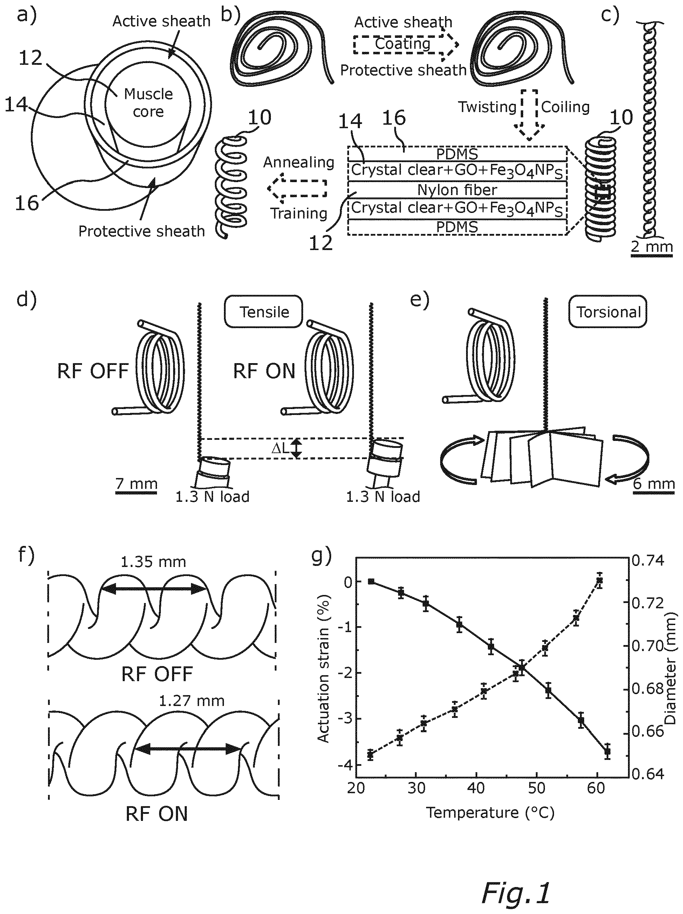

Fig. 1 shows the design, fabrication process, actuation modes and actuation mechanism of a magnetically heated coiled muscle structure 10 according to the invention. Fig. 1a shows a cross section of a schematic picture of the coiled muscle structure 10 before being coiled. Fig. 1b shows the fabrication process of the coiled muscle structure 10 including the steps of coating, twisting and coiling, annealing, and training. Fig. 1c shows a photo of the fabricated 1 mm-diameter coiled muscle structure 10. Fig. 1d shows the contractile actuation and Fig. 1e the torsional actuation modes of the coiled muscle structure 10 under RF magnetic heating. Fig. 1f shows photos of the structural changes of the coiled muscle structure 10 before and after RF magnetic heating. Finally, Fig. 1g shows the length and diameter changes of the trilayer precursor fiber 10 before coiling with different temperature conditions.

In this connection it is noted that in the cross section of Fig. 1a as well as of Fig. 1b one can clearly see the three layered structure 10 of the device according to the invention comprising a core 12, an active sheath 14 and a protective sheath 16.

Fig. 1b also shows that the core is made of a nylon fiber 18, while the active sheath comprises a resin (in this case Crystal Clear) 20 with graphene oxide platelets GO as well as a first type of magnetic particles 22 respectively embedded in the resin 20. The protective sheath 18 of Fig. 1b is made of an elastomeric material 24, i.e. for example PDMS.

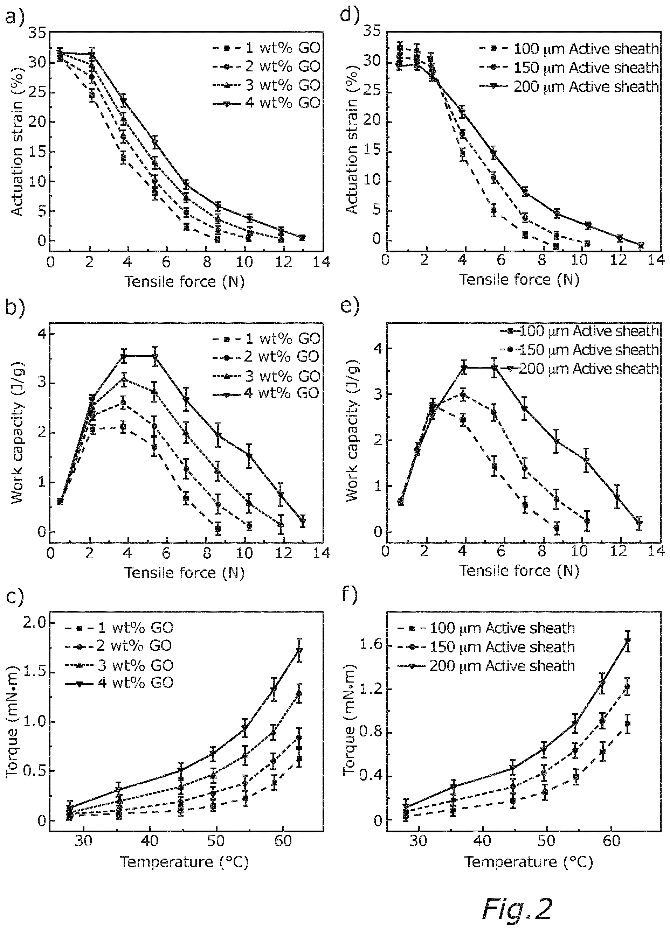

Fig. 2 shows the characterization results of the coiled muscle structure 10 performance. In Figs 2a and b one can see the influence of the GO platelet concentration, while Figs. 2c and d show the influence of the active sheath 14 thickness on the muscle actuation strain and work capacity under different tensile forces, respectively. The torque output of the coiled muscle 10 with different GO platelet concentrations and active sheath 14 thicknesses are shown in Figs. 2c and f, respectively.

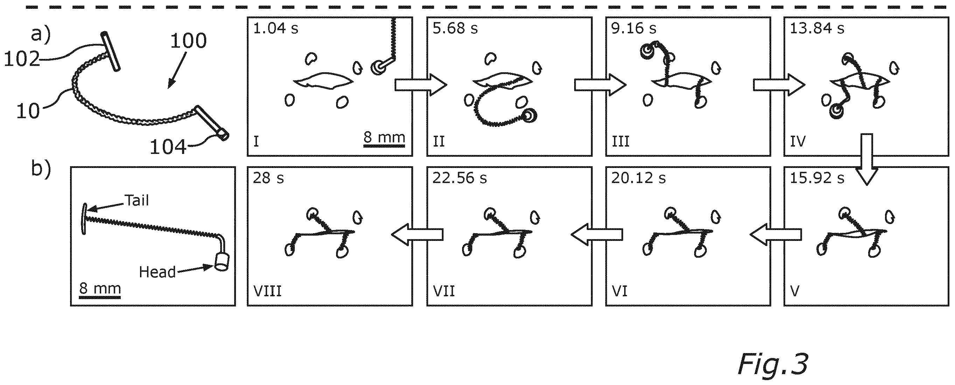

Fig. 3 shows a demonstration of a wireless suturing device 100. Fig. 3a shows a schematic including the design of the suturing device 100. Fig. 3b shows that the suturing device 100 consists of two printed obstacle panels 102, a coiled muscle structure 10, and a cylindrical NdFeB magnet 104. Fig. 3c shows that the wrapping motion of the suturing device 100 is controlled by the external magnetic field gradients-based pulling forces generated by an external permanent magnet 104 and the wound suturing process under RF magnetic heating.

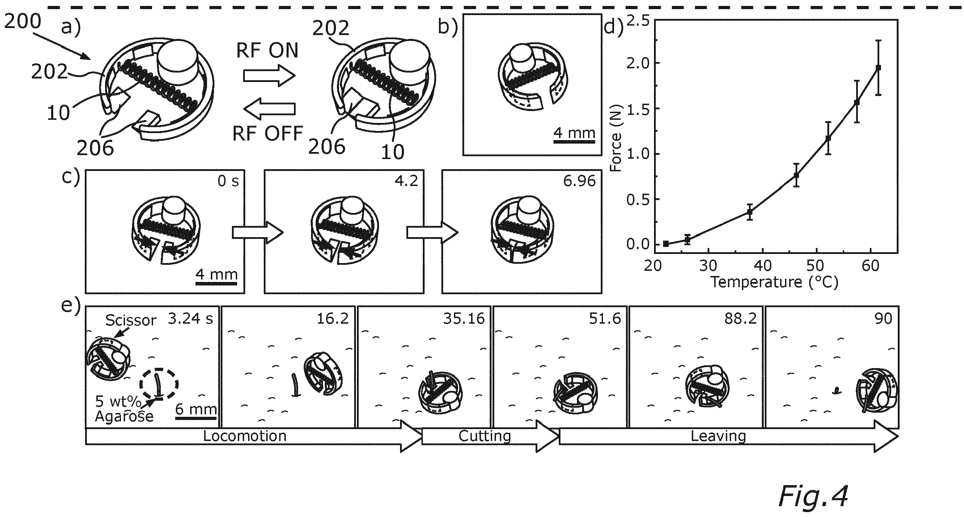

Fig. 4 shows a demonstration of a wireless medical scissor device 200. Fig. 4a shows a schematic including the cutting behavior of the designed scissor device 200. Fig. 4b shows an image of the scissor device 200 consisting of a 3D-printed polymer frame 202, coiled muscle actuator 10, magnetic part (NdFeB microparticles embedded inside an Ecoflex-30 silicone rubber matrix) 204, and two cutting blades 206. Fig. 4c shows the cutting behaviour of the scissor device 200 under RF magnetic heating. Fig. 4d shows the cutting force of the scissor device 200 measured during the cutting process. Fig. 4e is a video snapshots of the device 200 rolling on the surface by an external magnetic field rotation using a permanent magnet to reach a target agarose gel post and cutting the post cutting by RF heating.

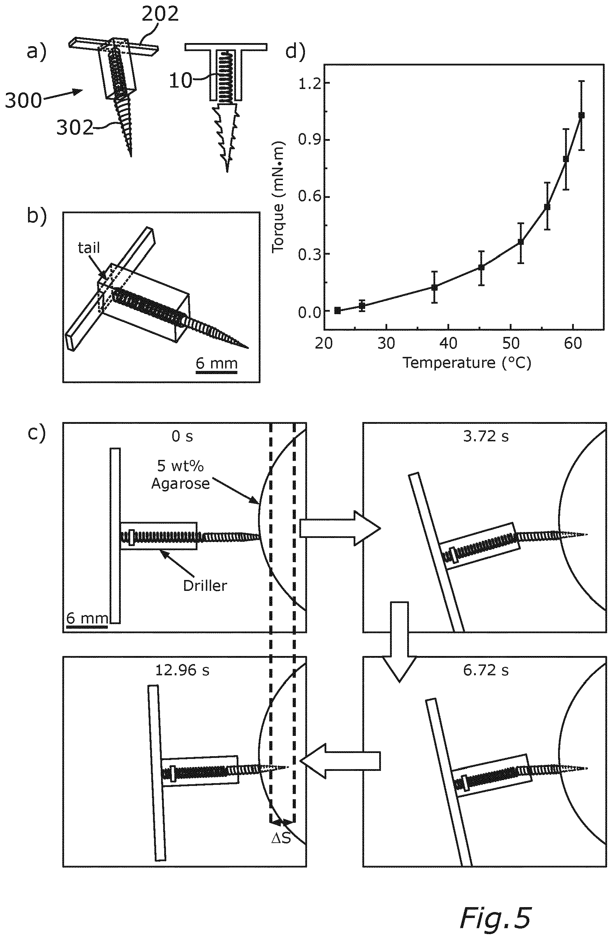

Fig. 5 shows a demonstration of a wireless medical driller device 300. Fig. 5a shows a schematic of the designed driller device 300. Fig. 5b is an image of the driller device 300 composing of the 3D-printed polymer frame 202 and the coiled muscle 10. Fig. 5c shows video snapshots of the device 300 drilling an agarose gel surface, resembling a soft biological tissue, for a distance, when magnetically heated. Fig. 5d shows the measured drilling torque of the device 300 under RF heating.

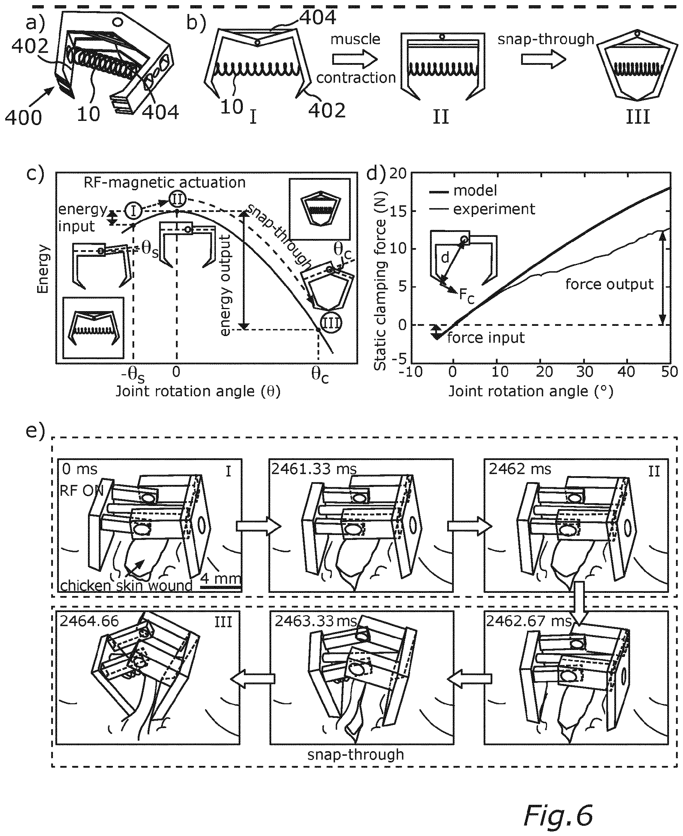

Fig. 6 shows a demonstration of a wireless bistable clamper 400. Fig. 6a shows a schematic of the clamper 400 designed with a bistable structure 10. The clamper 400 further comprises a body 402 that may, for example, be formed by the previously mentioned polymer frame, and a pretensioned elastomer 404. Fig. 6b is a sketch showing the clamping process under RF heating. Fig. 6c shows the energy distribution in the clamping process with different joint angles. Fig. 6d shows the experimental and the simulated clamping force of the clamper 400 with different designed joint angles. Fig. 6e includes images showing that the clamper device 400 clamps an ex vivo chicken tissue wound.

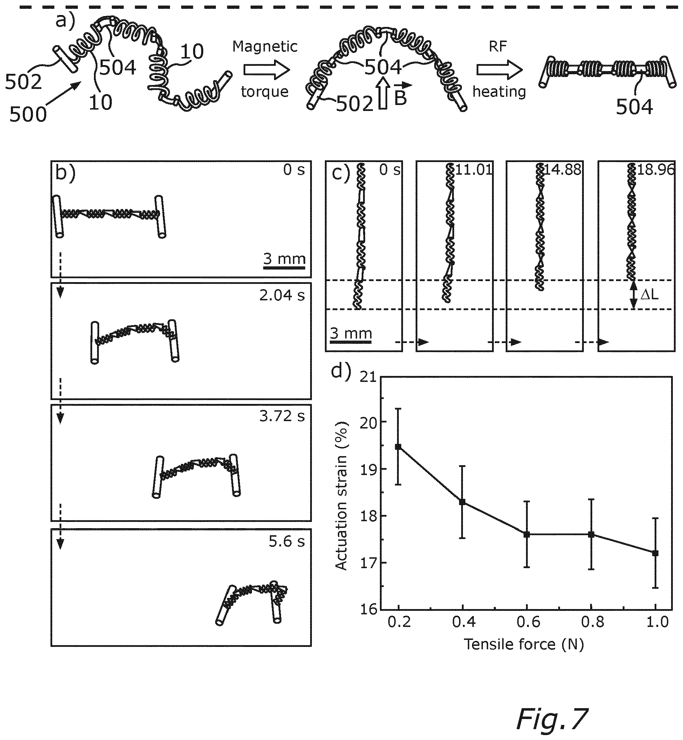

Fig. 7 shows a multi-linked coiled muscle device 500. Fig. 7a is a schematic showing the bending of the multi-linked coiled muscle device 500 under magnetic torque and the contraction behavior under RF magnetic heating. The multi-linked coiled muscle device 500 was encoded with a magnetization profile. That is, the coiled muscle structure 10 of the multi-linked coiled muscle device 500 comprises a fourth layer 26 with said layer being a magnetization sheath 26. The magnetization sheath 26 comprises a combination of a second type of magnetic particles 28 together with an elastomer 30. This multi-linked coiled muscle device 500 is composed of two bars 502 and four coiled muscle structures 10 linked by a connector 504. Fig. 7b shows images showing the "walking" behaviour of the multi-linked coiled muscle device 500 under magnetic torque and gradient. Fig. 7c shows video snapshots indicating the contraction performance of the same multi-linked coiled muscle device 500 under RF magnetic heating. Fig. 7d shows the actuation strain of this multi-linked coiled muscle device 500 under different tensile forces.

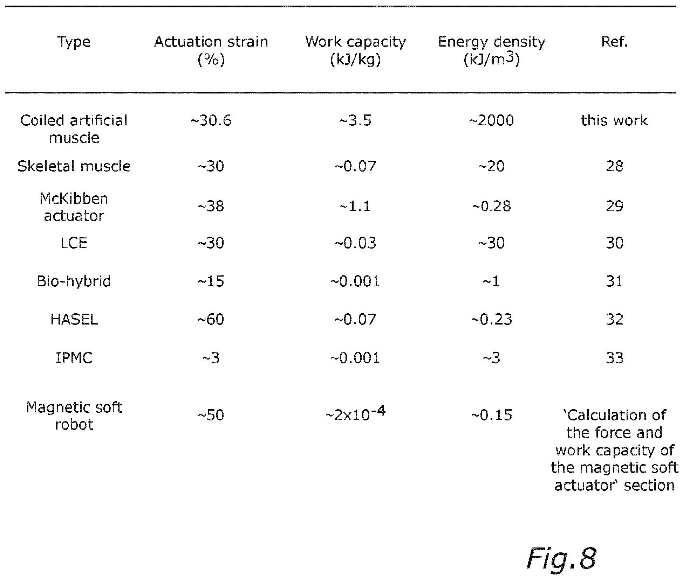

Fig. 8 includes Table 1 that shows a comparison of different types of soft and muscle actuators in terms of actuation strain, work capacity and energy density, wherein the expression "LCE" stands for "liquid crystal elastomer", "HASEL" stands for "hydraulically amplified self-healing electrostatic actuator" and "IPMC" stands for "ionic polymer-metal composites". It can be clearly seen that the magnetic soft robot, which is the device according of the invention, comprises a much higher actuation strain and work capacity than the other examples given in said table.

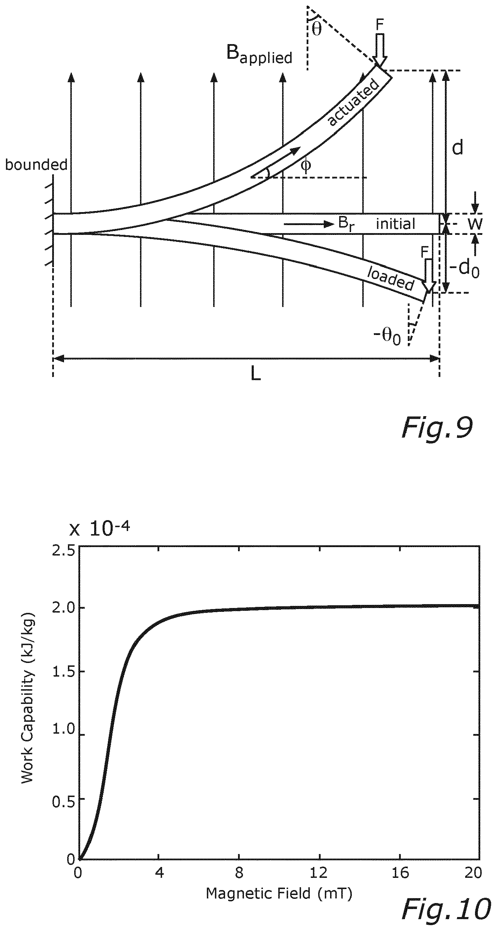

Fig. 9 shows a schematic of the actuation of a magnetic soft actuator 10 according to the invention. A detailed explanation of this Figure is given below.

Fig. 10 includes a diagram showing a calculation of the magnetic soft actuator 10 work capacity, wherein the theoretical work capacity of the magnetic soft composite actuator is a function of the applied magnetic field. A detailed explanation is given below.

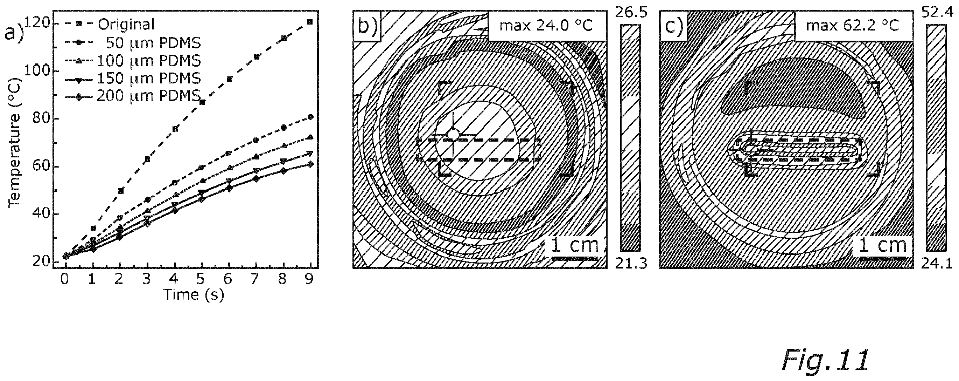

Fig. 11 shows the thermal characterization of the coiled muscle structure 10 according to the invention. Fig. 11a shows the surface temperature changes of the coiled muscle structure 10 with different coated PDMS thicknesses under RF heating. Infrared thermal images of the coiled muscle structures 10 with a PDMS sheath 16 thickness of 200 $\mu$m are shown in Figs. 11b and 11c before and after RF heating, respectively. In this connection it should be noted that the black dashed rectangle indicates the position of the coiled muscle structure 10.

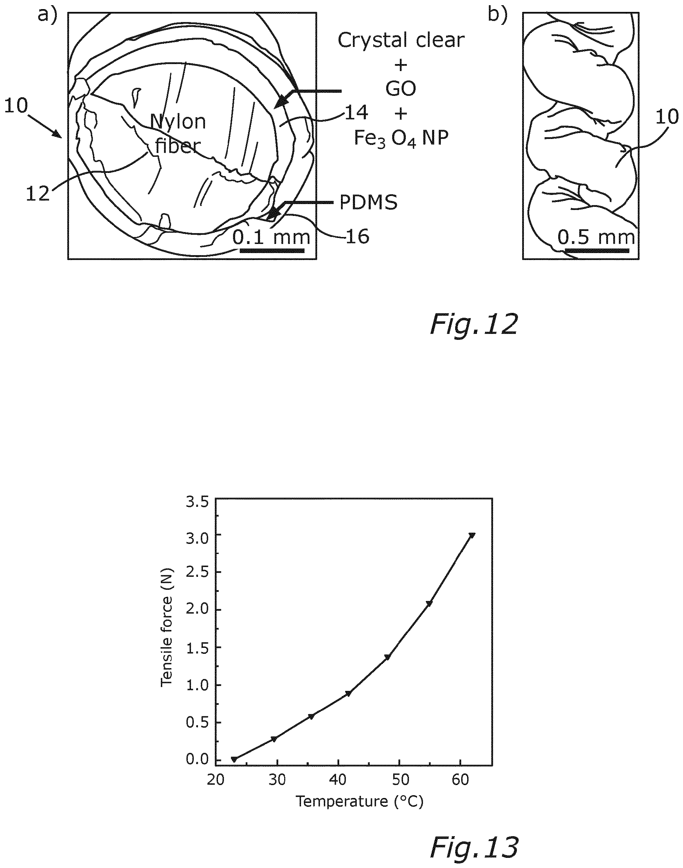

Fig. 12 includes a SEM morphology characterization, i.e. Fig. 12a shows a cross-sectional structure of the precursor fiber 10 and Fig. 12b shows the final coiled muscle structure 10.

Fig. 13 shows an output force characterization, wherein the tensile force of the coiled muscle 10 has a constant strain of 0.1% under heating.

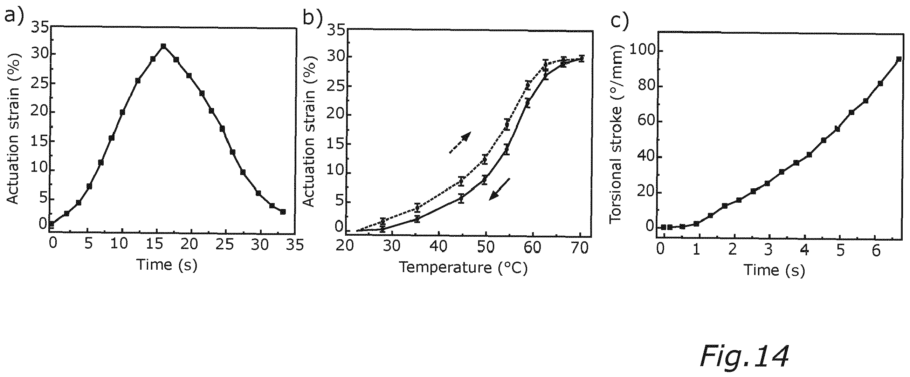

Fig. 14 shows the tensile strain and torsional stroke of the coiled muscle 10. The change of tensile actuation strain versus time and temperature in the contractile actuation is shown in Figs. 14a and 14b, respectively. Fig. 14c shows the torsional stroke in the torsional actuation. In this connection it should be noted that in Fig. 14b the red line and black line represent the temperature increasing and decreasing processes, respectively.

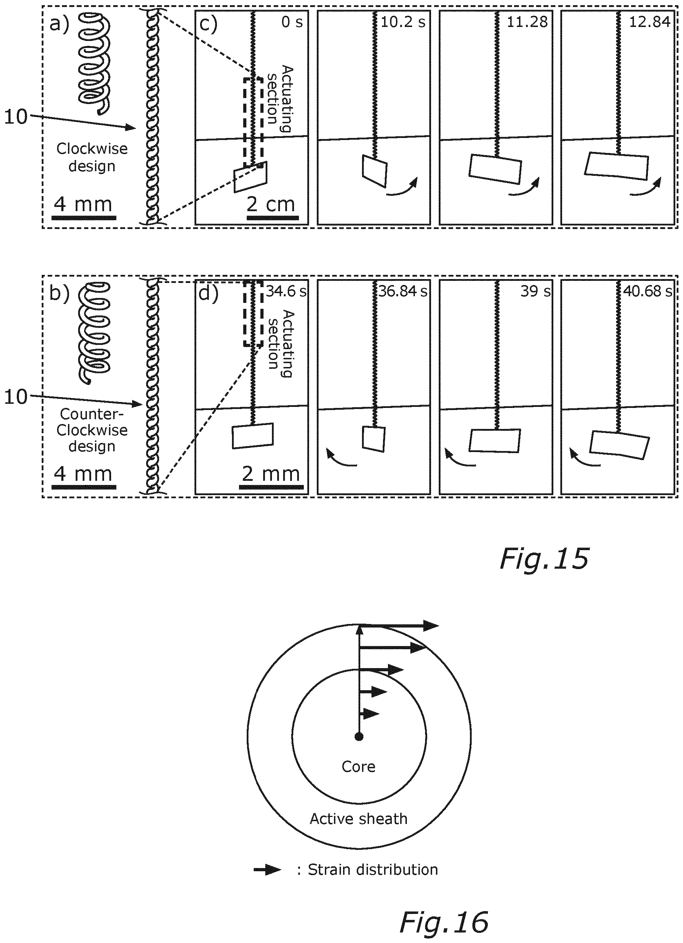

Fig. 15 shows a reversible torsional actuation. The lower clockwise section of the coiled muscle 10 is shown in Fig. 15a, Fig. 15b shows the upper counter-clockwise section of the coiled muscle 10. The corresponding counter-clockwise rotation and clockwise rotation of this coiled muscle 10 under RF heating is shown in Fig. 15c and 15d, respectively.

Fig. 16 shows a cross sectional view of a strain distribution of the coiled muscle 10. A detailed explanation of this Figure is given below.

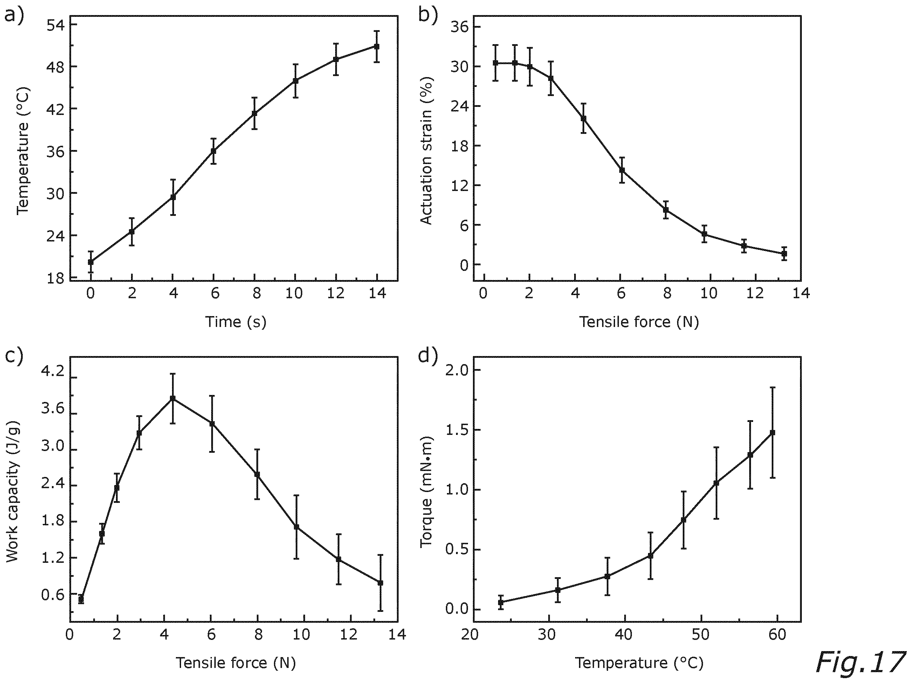

Fig. 17 shows a characterization of the coiled muscle performance in deionized water. In Fig. 17a one can see the temperature changes of the coiled muscle 10 under RF heating. Figs. 17b and c show the actuation strain and the calculated work capacity of the coiled muscle 10 with different tensile forces. Fig. 17d shows the torsional torque of the coiled muscle 10 under RF heating. In this connection it should be noted that the coiled muscle 10 on which the numbers of Fig. 17 are based is composed of 4 wt% GO and a 200 $\mu$m thick active sheath 14.

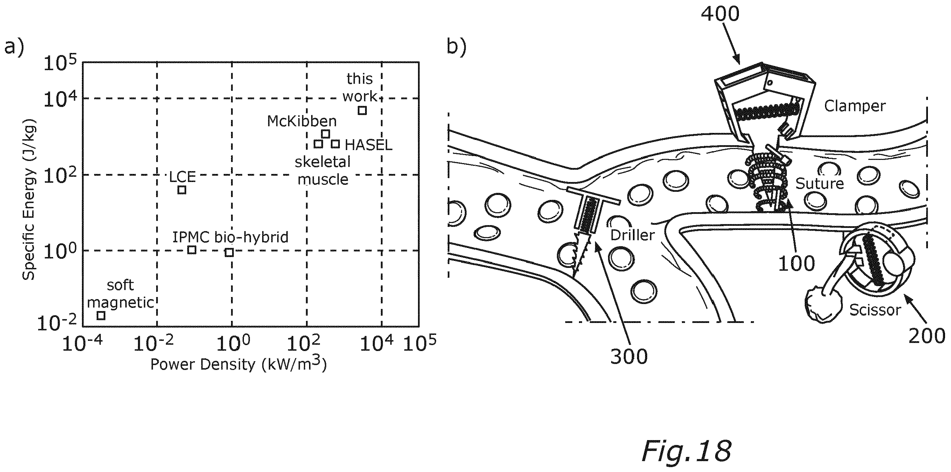

Fig. 18 shows the performance evaluation and application prospects of the coiled muscle 10. Fig. 18a shows a comparison of different types of soft actuators 100, 200, 300, 400 in terms of work capacity and power density. Fig. 18b includes a schematic showing the coiled muscle actuator engineered into a suturing device 100, scissor device 200, driller device 300, and a clamper device 400 toward biomedical applications.

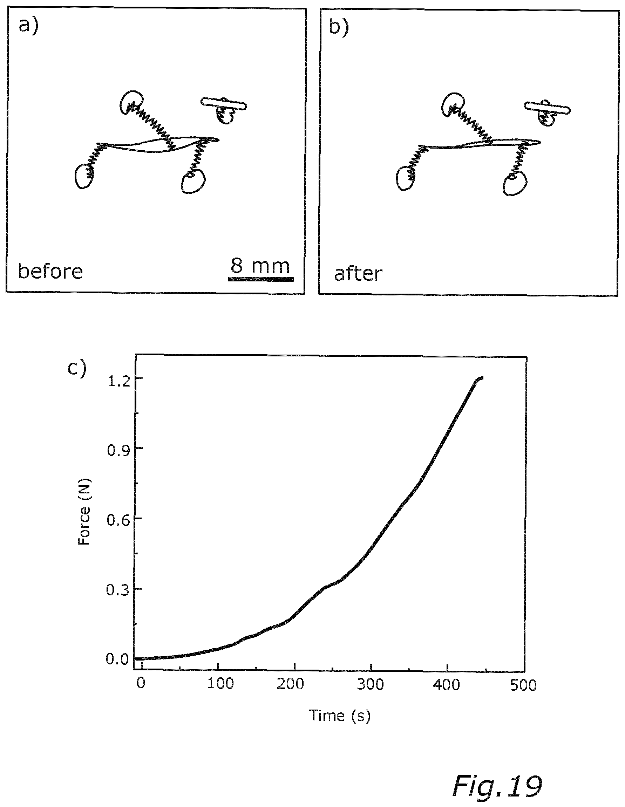

Fig. 19 includes force tests for the suturing device 100. Photos of a pigskin wound before and after closing by an Instron machine pulling are given in Figs. 19a and 19b, respectively. Fig.19c shows the pulling force for this pulling process.

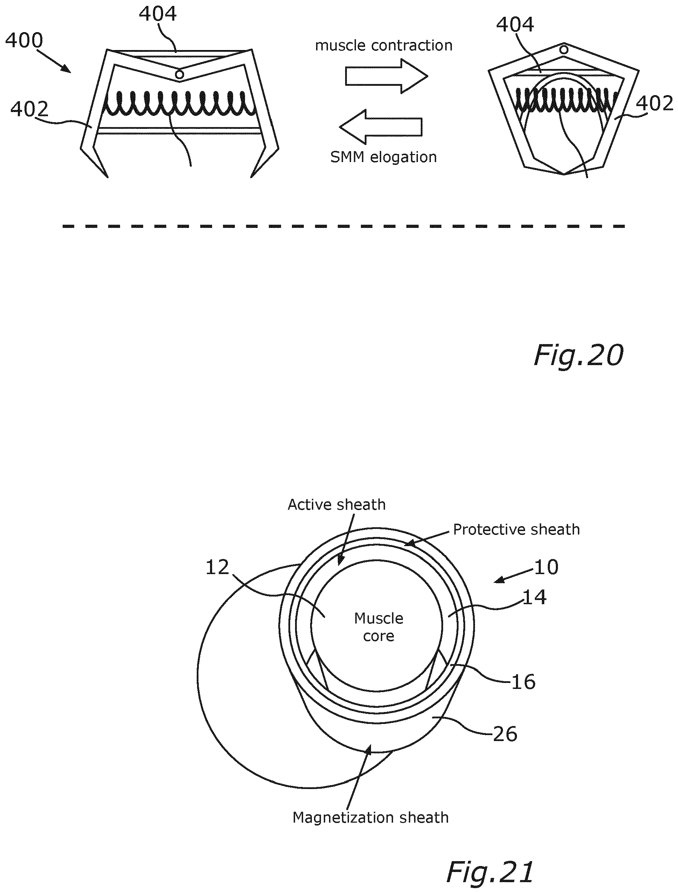

Fig. 20 shows a design of a reversible clamper device 400 including a schematic showing the reversible clamping process cooperated with the muscle contraction and shape memory material (SMM) elongation.

Fig. 21 shows a precursor fiber design of the multi-linked coiled muscle 500. The schematic shows the cross-sectional structure of the precursor fiber 10 after coating the magnetization sheath 26.

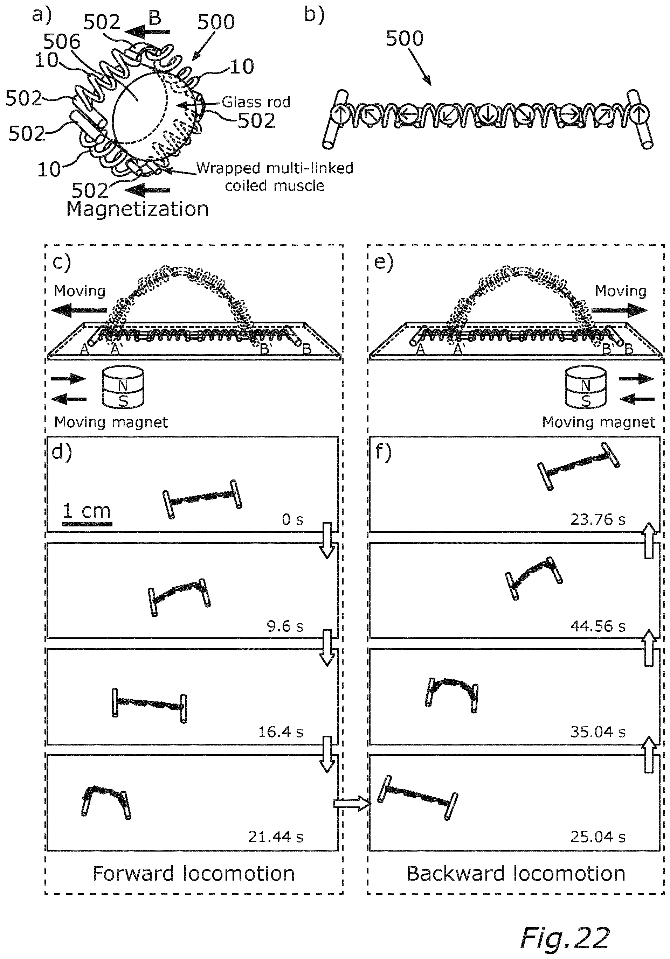

Fig. 22 shows a surface walking locomotion of the multi-linked coiled muscle 500. Schematic Fig. 22a shows a multi-linked coiled muscle 500 wrapped around a cylindrical glass rod 506 and magnetized by a uniform 1.8 T magnetic field. Fig. 22b shows the corresponding distribution of magnetization directions. Figs. 22c and 22d show a forward locomotion while Fig. 22e and 22f show a backward locomotion of the multi-linked muscle 500 under the external magnetic field.

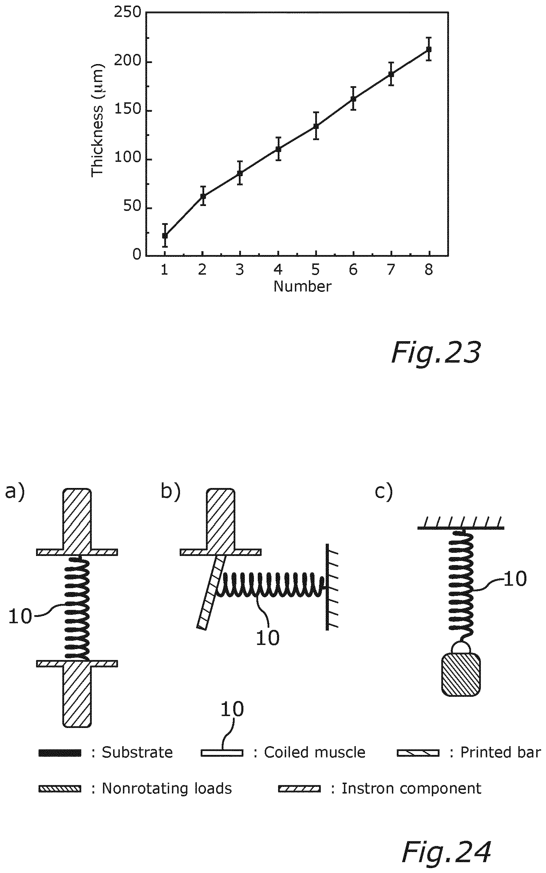

Fig. 23 shows the characterization of the thickness of an active sheath 14, wherein the thickness of the active sheath 14 is plotted versus the number of times the core 12 has been coated with the droplet-coating technique.

Fig. 24 is a schematic of the measurement setup for the measurement of the tensile actuation force (Fig. 24a), the torsional torque (Fig. 24b), and the actuation strain (Fig. 24c).

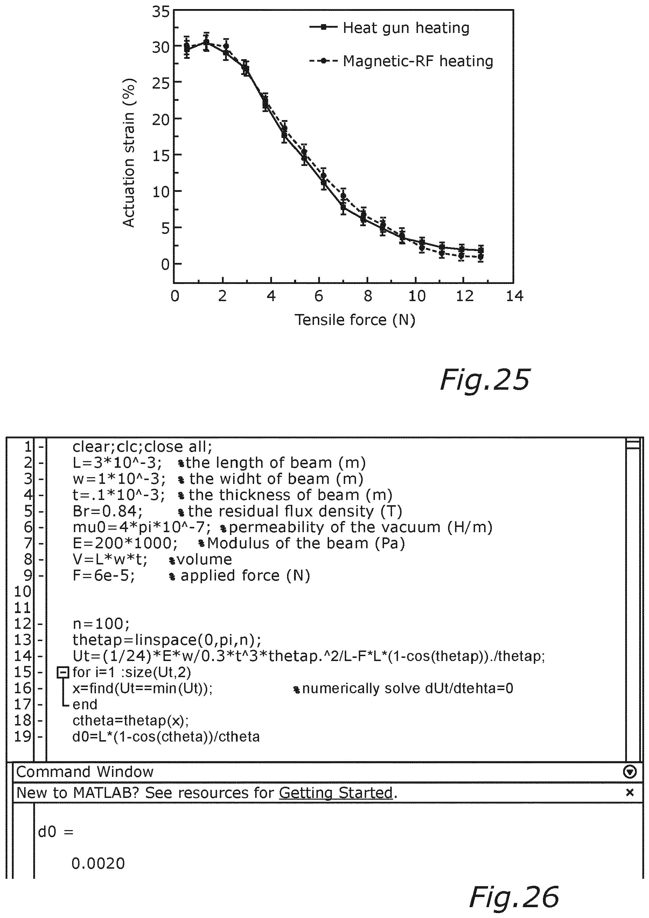

Fig. 25 shows the measurement results of the actuation strain measured with different methods and a comparison of the coiled muscle actuation strain under various loads between the heat gun heating and the RF heating.

Fig. 26 shows the Matlab code for the calculation of the output force of the magnetic soft actuator 10. The Matlab code calculates the output force of the magnetic soft actuator 10 by using a numerical method.

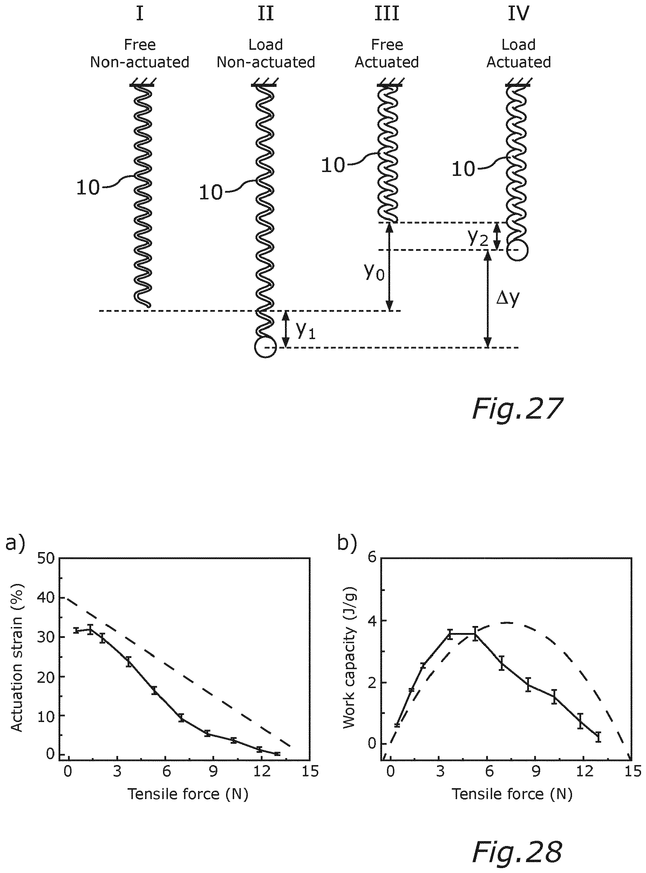

Fig. 27 shows how the actuation strain and work capacity of the coiled muscle 10 can be calculated. The schematic indicates the dependence of the length on the loading force for the actuated and the non-actuated coiled muscle 10.

Fig. 28 shows a comparison of a theoretical and an experimental coiled muscle 10, wherein in Fig. 28a one can see the comparison of the actuation strain, while Fig. 28b shows the comparison of the work capacity results under different loading forces. The theoretically calculated results are represented by a red dash line while the experimentally measured results are represented by a green solid line. In this connection it should be noted that the experimental results are based on a coiled muscle 10 composed of 4 wt% GO and 200 $\mu$m active sheath 14 thickness.

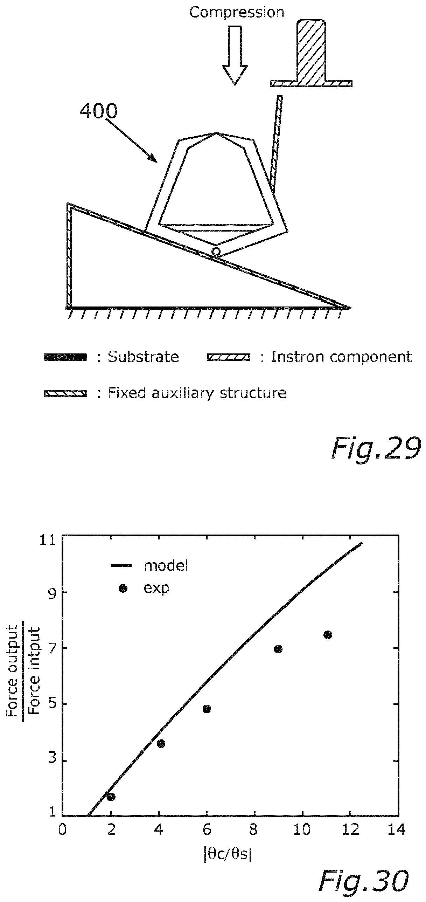

Fig. 29 shows a clamper device 400 including the setup used to test the clamping force of the bistable clamper 400.

Fig. 30 shows the optimization results of the bistable design by showing the ratio between the output force and the input force as a function of $\theta c/\theta s$.

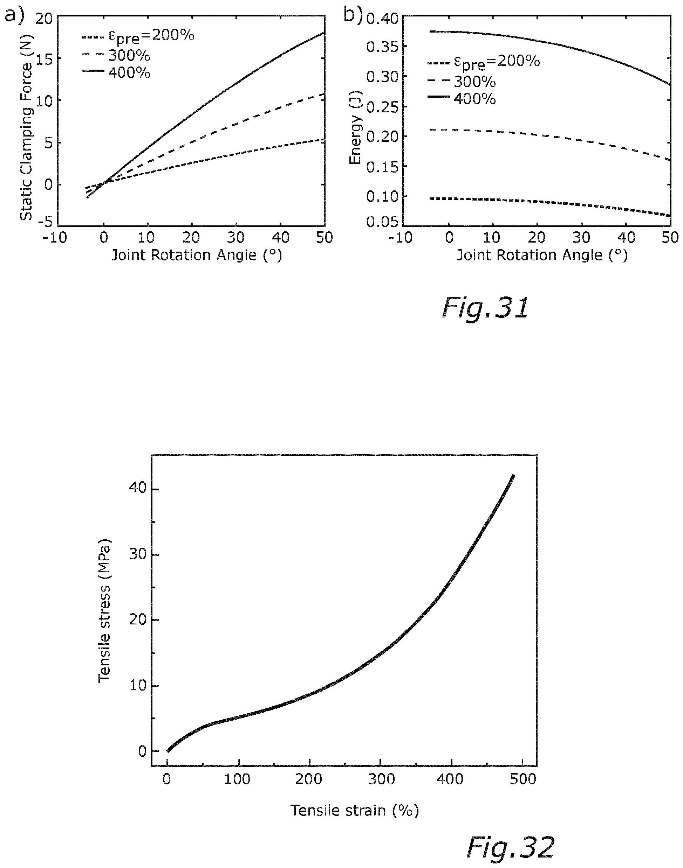

Fig. 31 shows the static clamping force and potential energy of the bistable clamper 400. Fig. 31a shows the theoretical static clamping force versus the joint rotation angle for different pre-stretched strains in the elastomer 404 while Fig. 31b shows the theoretical potential energy versus the joint rotation angle for different pre-stretched strains in the elastomer 404.

Fig. 32 includes a characterization of a pre-stretched elastomer 404 and the tensile stress-strain curve of the elastomer 404 used for the bistable clamper 400.

In the following, different embodiments of the invention that have already been realized as well as corresponding experimental setups, calculations and results are described in detail.

**Device design, fabrication and mechanism**

The design of the proposed structure 10 (hereinafter muscle actuator 10) is shown in Fig. 1a. It consisted of three layers: a high-strength nylon fiber as the muscle core 12 that provides the restoring stiffness, a protective sheath 16 at the outermost surface to insulate the inner layers both mechanically and thermally to and from the external environment (see Methods-section below and Fig. 11), and an active sheath 14 between the aforementioned core 12 and protective sheath 16. The active sheath 14 was composed of three elements: Crystal Clear™ matrix 20, a rigid urethane casting resin 20, which is a heat-cured commercial product, superparamagnetic $Fe_3O_4$ nanoparticles 22 and mechanical strength-enhancing GO platelets. The trimorph structure was finally twisted to form the muscle structure 10, i.e. the coiled muscle 10.

In this connection it should be noted that the nylon fibers have a strength selected in the range of 1.0 to 1.2 GPa.

The three layered structure 10 was fabricated through a customized droplet-coating technique (Fig. 1b). As the first step, a 0.45 mm-diameter nylon fiber 12 was dipped into the uncured Crystal Clear™ resin 20 solution, which is mixed with $Fe_3O_4$ nanoparticles 22 and GO platelets, to create the active sheath 14. Next, it was coated with PDMS 24 using a similar droplet-coating process to form the protective sheath 16. After the PDMS layer 16 was fully cured, its cross-sectional structure was characterized by scanning electron microscopy (SEM), as shown in Fig. 12a. The thickness of each sheath 14, 16 can be controlled by repeating the droplet-coating method multiple times. Then, this final coiled structure 10 was obtained by continuously twisting the above trimorph structure 10 to form the coiled structure 10 first and then annealing and training it. As shown in the optical microscopy image (Fig. 1c) and the zoomed SEM image (Fig. 12b), the resulting muscle 10 has a coiled shape, which is approximately 1 mm in diameter.

The coiled muscle 10 was wirelessly activated by heating the embedded magnetic nanoparticles 22 through an external RF magnetic field generator. The coiled muscle 10 had two operation modes under wireless RF-magnetic heating. First, when both ends are allowed to move axially but constrained from rotation, it exhibited contractile actuation. As shown in Fig. 1d, it could pull a 1.3 N target with a strain of 30%. It could even pull up to 12.6 N with a reduced strain (1%), which is discussed later in connection with Fig. 2a. As shown in Fig. 13, the tensile force output of the coiled muscle 10 with a constant strain of 0.1% could reach ~3.1 N.

Second, when one end of the coiled muscle 10 was fixed and it was free to rotate, it exhibited torsional actuation, as shown in Fig. 1e, where its torque could reach approximately 1.76 mN·m. In addition, the change of actuation strain and torsional stroke in this contractile and torsional actuation was characterized, respectively (Fig. 14). It indicated that the contractile actuation is a reversible actuation, while the torsional actuation is a one-time actuation only. This is because that there is no constraint during the torsional actuation, which releases the strain energy generated in the twisting and coiling fabrication process. To reverse the torsional actuation, the coiled muscles 10 were designed in two sections with different twisted directions. This design is shown in Fig. 15. By actuating the lower section, the actuator rotates counter-clockwise, opposite to its clockwise design. By actuating the upper section, the actuator rotates clockwise.

The proposed coiled muscles 10 demonstrate new characteristics that are inaccessible to previous twisted muscles, including magnetic responsiveness, enhanced mechanical output and better compatibility with biological tissues, thereby enabling wireless medical device applications. Its actuation mechanism is due to the untwisting process of the fiber caused by thermal contraction of the fiber in the longitudinal direction and thermal expansion in the radial direction. This mechanism was studied by recording the actuation process under an optical microscope. As shown in Fig. 1f, there was a contraction between the coil pitch from 1.95 mm to 1.77 mm before and after RF magnetic heating. The length and the diameter of the trimorph structure 10 before the twisting and coiling process under different temperatures was also characterized. As indicated in Fig. 1g, there was a contraction in the axial direction of 3.7% and an expansion in the radial direction of around 11.3%, further verifying the actuation mechanism.

The key feature of this trimorph design lies in the composition of the active sheath 14 (the intermediate layer), which includes the resin matrix 20, magnetic nanoparticles 22 and GO platelets. First, Crystal Clear™ resin 20 was used as the matrix because of its intrinsically high toughness and large expansion ratio. Both were beneficial in delivering high stroke and high mechanical output. Second, $Fe_3O_4$ nanoparticles 22 enabled the magneto-thermal effect, making it possible to wirelessly actuate the muscle 10 by an RF-magnetic field. In this connection it should also be noted that the $Fe_3O_4$ nanoparticles 22 themselves also played a role in toughening the muscle 10. Third, GO platelets, substantially aided the fiber strain energy storage due to their unique 2D geometry, especially in the case of being twisted much more than of nanoparticles or carbon-nanotubes as toughening agents. In this embodiment, the GO platelets were introduced into the active layer 14, which is located near the outermost surface, because the expansion force was more effective when they were acting near the margin of the yarn (Fig. 16), where the strain energy is at its maximum.

**Performance characterization**

The performance of the magnetically-heated coiled muscle 10, such as its actuation strain and work capacity and torque output, was highly dependent on the GO platelet concentration and the active sheath thickness. These two factors were systematically characterized in Fig. 2. First, Fig. 2a-c show the results of increasing the platelet concentration from 1 wt% to 4 wt% while fixing the active sheath thickness at 200 μm. 4 wt%-concentrated coiled muscles 10 delivered a higher actuation strain. The load-optimized work capacity and torque increased from 2.46 J/g and 0.66 mN·m to 3.5 J/g and 1.76 mN·m by tuning the toughening agent from 1 wt% to 4 wt%, respectively. This is because the GO platelets exhibited substantial bending and twisting behavior inside the fiber, which improved the stored mechanical energy during twisting and coiling.

Second, Fig. 2d-f show the result of increasing the sheath thickness from 100 to 200 μm while fixing the GO platelet concentration at 4 wt%. Fig. 2d shows the result of the actuation strain. When the load was less than 2.1 N, the coiled muscle 10 with a thicker sheath thickness had a lower actuation strain. This was because of the intercoil contact. When the load was higher than 2.1 N, the coiled muscle 10 with a thicker active sheath 14 thickness had a higher actuation strain. Fig. 2e and 2f show that the load-optimized work capacity and torque could respectively increase from 2.19 J/g and 1.01 mN·m to 3.5 J/g and 1.76 mN·m by increasing the active sheath thickness from 100 to 200 μm. Such a result agrees with the previous literature findings, where thickening of the outer layer, rather than the core, is much more effective in improving the artificial muscle performance. Note that the active sheath 14 tended to break in the experiments when the platelet (GO) concentration and the active sheath 14 thickness exceeded 4% and 200 μm, respectively. Therefore, no further measurements were conducted beyond these parameters. In addition, the coiled muscle 10 was characterized in deionized water. The results include surface temperature, tensile actuation strain, tensile work capacity and torsional torque, which are shown in Fig. 17. By comparing with Fig. 2, the output performance of the coiled muscle 10 inside the water is similar to that in the air. This could be attributed to the insulation of the protective sheath 16.

Finally, the mechanical performance of the proposed coiled muscles 10 was further compared with other types of soft actuators, as summarized in the 'Calculation of the force and work capacity of the magnetic soft actuator' section, Fig. 18a and Table 1 below. The coiled muscle 10 has a higher work capacity than popular soft actuators, including the skeletal muscle, McKibben actuator, liquid crystal elastomer (LCE), bio-hybrid actuator, hydraulically amplified self-healing electrostatic actuator (HASEL), ionic polymer-metal composites (IPMC) and magnetic soft actuators (see 'Calculation of the force and work capacity of the magnetic soft actuator' section below). In the category of magnetic soft actuators, the magnetically-heated coiled muscles 10 exhibit about $2 \times 10^5$ times higher actuation force output than the previous magnetic soft actuators and $\sim 1.75 \times 10^4$ times higher work capacity, greatly improving the mechanical performance of the magnetic soft actuators and expanding their capabilities to medical device applications requiring high force and work density output.

**Application demonstrations**

Using the given magnetically-heated coiled muscle prototype 10, several proof-of-concept demonstrations towards wireless medical device applications could be reported (Fig. 18b). The first demonstration shows a suture device 100 (Fig. 3). Suturing is a critical aspect of most surgeries. Conventional suturing relies on articulated tools and physical manipulation of the suturing device. Due to the large footprints of the manipulation tools, conventional suturing usually takes large surgical invasiveness, which will cause more tissue damage, longer recovery times, or other associated infections. Here, a wireless suture medical device 100 performing wound suturing on a pigskin *ex vivo* is demonstrated. This device 100 was possible due to the precise magnetic field control and large output force of the coiled muscle 10.

As shown in Fig. 3a-b, the suturing device 100 was composed of two bars 102 on the two sides of the coiled muscle 100 and a magnetic part 104, which was a cylindrical NdFeB magnet 104, at the forefront. The bars 102 were made by the pure nylon fiber utilized to prevent the rotation of the coiled muscle 10 under RF-magnetic heating. It also acted as an anchor during the contraction of the coiled muscle 10 to enclose the wound. The front NdFeB magnet 104 was used to pull and guide the coiled muscle 10 in wrapping around the wound by the gradient pulling of an external permanent magnet. Note that the $Fe_3O_4$ nanoparticles 22 used in the active sheath 14 were too weak to generate enough pulling force through this magnet field generated by a permanent magnet.

The whole suturing process is shown in Fig. 3c, which was divided into two steps. In the first step, the suturing device 100 was wrapped around the wound under the magnetic field (I-V in Fig. 3c), which demonstrated the flexibility (small bending stiffness) of the coiled muscle 10. In this connection it is noted that holes were made on the pigskin beforehand to facilitate easier piercing. Due to the high stiffness of the porcine skin, currently, it is not possible to penetrate the tissue even though a suture needle on one end of the actuator was added. However, this penetration could be achieved by using a sharper needle or a greater magnetic gradient force. In the second step, the RF-magnetic heating was turned on to contract the artificial muscle 10 for wound closure (VI-VIII in Fig. 3c), indicating the large output force of the coiled muscle 10.

A control experiment was done by using an Instron tensile measurement device to pull the head of the device to close the wound. It required a force of 1.21 N with 29.6% actuation strain to realize the same effect (Fig. 19). Therefore, this demonstration indicates that the proposed coiled muscle 10 exhibits both high flexibility and large output force. When RF-magnetic heating was turned off, the recovery of the contracted muscle 10 could reopen the wound. However, little reopening was observed. This could be due to the friction between the pigskin and the substrate where the pigskin was attached to. This wound reopening issue can be solved by making a proper knot or by designing an anchoring structure, such as a bistable structure, at the end of the device 100 in the future. In addition, the device 100 is intended to remain in the body until the wound recovers. Currently, the outermost protective layer 16 of the device is composed of PDMS, which is a biocompatible material. However, further biocompatibility has to be tested to justify the overall biocompatibility of the device 100. At last, how to release the device 100 after the wound is healed is worth of further investigation.

In the second demonstration shown in Fig. 4, a scissor medical device 200 was designed. This scissor function is surgically important for cutting the epidermis or internal biological tissues. Tetherless scissors can potentially reach confined *in vivo* sites and implement the above functions. As shown in Fig. 4a-b, a scissor medical device 200 is shown consisting of a circular printed frame 202, magnetic parts 204 (as a composite of NdFeB microparticles embedded inside a silicone elastomer (Ecoflex 00-30) matrix and two metal blades 206 assembled with the coiled muscle structure 10. The circular shape 202 and NdFeB magnetic composite parts 204 were used to enable the rolling locomotion of the device 200 on solid tissues under the control of an external permanent magnet, which is teleoperat-ed. The blades 206 were carefully designed not to impede the rolling motion and hurt the surrounding tissue during the locomotion. Under RF-magnetic heating, this scissor device 200 exhibited cutting behavior based on the contraction of the coiled muscle 10 (Fig. 4c).

The cutting force of this scissor device 200 was quantitatively characterized as shown in Fig. 4d. The force increased with the heating temperature, and the maximal force was ~2 N. In Fig. 4e, the feasibility of the scissor device 200 was demonstrated by cutting a synthetic gel pillar toward cutting tissues in future medical applications. After being rolled to the workplace with a manually rotating magnetic field by using an external NdFeB permanent magnet (locomotion part in Fig. 4e), the device 200 is carefully controlled to orient its blades on a gel pillar made of 5 wt% agarose (a synthetic tissue-like material). Next, RF-magnetic heating was turned on to activate the artificial muscle 10 and eventually cut the pillar in half (cutting part in Fig. 4e). After cutting, the scissor device 200 could be driven away from the agarose tissue under similar magnetic field control with the locomotion part (leaving the scene part in Fig. 4e).

Third, a wireless medical driller device 300 using torsional actuation of the coiled muscle 10 under RF-magnetic heating was demonstrated (Fig. 5). A wireless driller is a promising device for medical applications including navigation, clots removal or tissue biopsies as the treatment procedure is untethered, precise and minimally invasive. However, one challenge of these wireless drillers is to achieve large output torques. For example, the output torque of previously reported magnetic drillers was only 149 $\mu$N·m. The driller device 300 according to the invention adopted a new mechanism that leveraged the high energy-releasing capability of the coiled muscle structure 10 during the untwisting process to achieve a large torque output to overcome these challenges. As can be seen in Figs. 5a-b, the coiled muscle structure 10 was assembled with a screw 302 at the bottom end and a chassis 202 with a rectangular tail. The rectangular tail of the chassis 202 was tethered on one side of the coiled muscle structure 10, which was used to limit the rotation of one end of the muscle structure 10 during the drilling process. The device 300 could drill into the 5 wt% agarose gel under RF-magnetic heating (Fig. 5b) as a synthetic tissue-like material for drilling demonstration. The drilling displacement was about 3 mm, as indicated by $\Delta S$ in Fig. 5c, and the drilling torque could reach about 1.2 mN·m (Fig. 5d). Currently, the device 300 needs to rest on another structure to function, as the tail chassis needs to counteract the torque being applied by the screw drilling. The reaction force can be distributed in a wider area by designing a larger tail, with a consideration of the maximal pressure that can be supported by the targeted environment.

In the fourth case, a wireless bistable clamper device 400 (Fig. 6) is demonstrated. A clamper is useful for wound clamping and tissue pinching in surgical operations. The clamper device 400 is constructed by combining a bistable linkage structure 402, 404 with the proposed magnetically-heated coiled muscle structure 10. The bistable structure acts as a force amplifier, which can further boost the output force, response speed, and actuation strain performance of the twisting muscle structure 10. The design is shown in Fig. 6a, and the actuation mechanism is shown in Fig. 6b. The fabrication details are shown in the methods section. A theoretical model was built to guide the clamper design (see below). The mechanical energy of the clamper was stored in a pre-stretched elastomer embedded within the rigid linkage (Fig. 6a, b), which could be tuned from low to high energy storage capacity through simple elastomer pretension 404 or setting the stop angle in the linkage.

This pre-stretched elastomer 404 enabled two stable states (state I and state III in Fig. 6c) for the clamper 400. When RF-magnetic heating was turned on, the clamper 400 deformed towards the unstable state II, where the elastomer 404 stored the most energy. Once it bypassed the unstable state, it rapidly snapped to the other stable state III. The coiled muscle structure 10 selected for this design had a force exertion of 1 N. As shown in Fig. 6d, the output force of the coiled muscle 10 could be amplified to 14 N with the proposed bistable design. A more detailed explanation of the bistable design can be found in the 'Modeling of the bistable clamper' section of this disclosure. By using such a design enhanced by the bistable structure, an *ex-vivo* clamping of a wound on a chicken skin could be demonstrated (see Fig. 6e). The process can be divided into two parts: muscle contraction and elastomer snap-through. When RF-magnetic heating is turned on, muscle contraction starts, driving the clamper 400 from stable state I to unstable state II, followed by a rapid snap to stable state III within 2.7 ms.

Furthermore, the bistable structure confers the design with two additional capabilities. First, the snap-through of the bistability enables much faster actuation than the coiled muscle structure alone. Second, the structure is also used to amplify the actuation strain of the muscle structure. Therefore, such a combination enables a wider design space for future miniature (medical) device applications. According to the embodiment shown, the clamping device is a one-time actuation device due to the presence of the bistable structure. However, the target function of this clamper device 400 is wound clamping and tissue pinching. Therefore, in most cases, this clamper 400 does not need to be reopened. Even commercial tethered clampers (such as SureClip™ Hemoclips) exhibit one-time actuation. To achieve a reversible function, an additional actuator can be introduced, such as a shape memory material, to switch the device 400 back to the open state (as shown in Fig. 20).

In the last embodiment, a multi-linked coiled muscle structure 500 with both programmable magnetic deformations and high force output (Fig. 7) is shown. To encode the magnetization profile on the coiled muscle structure 10, first, an additional sheath 26 is coated on the outer surface of the precursor fiber. This magnetization sheath 26 is composed of PDMS and NdFeB microparticles. With this modification (Fig. 21), the artificial muscle structure 10 has a total of three sheaths. In this connection it is pointed out that the protective sheath 16 is essential as it binds with the magnetization sheath 26 better than the active sheath 14 (Crystal Clear™). After the artificial muscle structures 10 were made, four pieces (~ 4 mm) of them were linked in series by a connector 504, as shown in Fig. 7a. The connector 504 is composed of PDMS and NdFeB microparticles. The connector 504 is softer than the artificial muscle structure 10 and therefore can amplify the magnetic deformation. At last, two bars 502 are added to both sides of the multi-linked coiled muscle structure 500 to prevent a rotation of the coiled muscle structure 10 under RF-magnetic heating. As shown in Fig. 7a and Fig. 22, after magnetization, the multi-linked coiled muscle 500 can realize deformation under external magnetic torque.

By using both magnetic torque and magnetic gradient, this multi-linked coiled muscle 500 exhibits a "walking" behavior, as shown in Fig. 7b. The moving direction of this multi-linked coiled device 500 can be controlled by the external magnetic field. It can move both forward and backward (Fig. 22). In addition, when both sides are constrained from rotation but allowed to move axially (Fig. 7c), this multi-linked coiled muscle 500 also exhibits contraction behavior with 19.5% actuation strain ($\Delta L$) under 0.2 N load force. The maximum tensile force of this coiled muscle structure is about 1 N with 17.3% actuation strain. The maximal force is limited by the NdFeB-PDMS connector as it breaks when the tensile force exceeds 1 N. With these capabilities, the coiled muscle can potentially realize more diverse manipulation behaviors and medical functions in addition to its high force output and work capacity.

**Discussion and Conclusions**

The coiled muscle structure 10 according to the invention can be improved in several directions. First, the current core 12 used for the above experiments is a commercially available nylon wire. It limits the potential to make miniature actuators. To address this drawback, electrospinning can be used to reduce the diameter of the nylon core 12 for a miniature actuator.

Second, the surface temperature of the coiled muscle structure 10 can be further tuned for different applications. However, effects of the elevated temperature depend on the specific application. For the current coiled muscle structure 10, a protective insulation sheath 16 on the outermost layer of the precursor fiber has been designed by using PDMS.

As shown in Fig. 11, the surface temperature was reduced by increasing the thickness of the protective sheath 16. The surface temperature of the current coiled muscle structure 10 was reduced to below 60°C. Additionally, the surrounding temperature quickly drops with increasing distance from the coiled muscle structure 10. As shown in Fig. 3c, the temperature drops to 30°C at a 2 mm separation from the coiled muscle structure 10.

In general, normal cells can withstand temperatures of up to 42-45°C. For the scissor 200, driller 300 and clamper 400 devices, however, due to the presence of the printed frame 202, the coiled muscle structure 10 is not in direct contact with the tissue.

As shown in Figs. 4, 5 and 6, the distances between the coiled muscle structure 100 and the tissue are approximately 5 mm, 4 mm and 1 cm for the scissor 200, driller 300 and clamper 400 devices, respectively. Thus, the temperature on the tissue is lower than 30°C, causing no damage to said tissue. For the suturing device 100 and the multi-linked device 500, the target application is to close a wound, such that the device 100, 500 is in direct contact with the tissue. In this scenario, the high temperatures may benefit the wound closure during the suturing process through hyperthermia. Such a topic is worthy of further investigation in the future. At last, if there is a demanding requirement to further reduce the surface temperature of the coiled muscle 10, it can be achieved by increasing the thermal insulation efficiency of the protective sheath 16. The thermal conductivity of PDMS elastomer is 0.16 W/mK, which can be switched by cellulose (thermal conductivity is 0.04 W/mK) or polyurethane (thermal conductivity is 0.032 W/mK) (54).

Third, although the fast response is not essential in the proof-of-concept application demonstrations, such as suturing 100, scissor 200, driller 300, clamper 400, and multi-linked actuator 500, it could be desirable for some future applications. For the current coiled muscle structure 10, the response speed can be increased by choosing materials with a lower glass-transition temperature, such as polycaprolactone. For these materials, an external insulation sheath (protective sheath) may no longer be required, thereby increasing the efficiency of heat dissipation.

## Materials and Methods

### Precursor fiber fabrication

The precursor fiber 10' can be and was fabricated by the so-called droplet-coating technique. In detail, 640 mg of $Fe_3O_4$ nanoparticles 22 (50-100 nm particle size, bought from Sigma-Aldrich company) and 63 mg of GO (Sigma-Aldrich company) were first added into 1.5 g mixed Crystal Clear™ 202 resin 20 (Smooth-On company, the initial modulus (at $\varepsilon$=1%) after annealing was measured to be 1.04 GPa; the volume expansion ratio was 29.5% at 120 °C). Its base and curing agent (w/w 10:9) are stirred for 1 min. Next, the above mixture was degassed for 5 min. Then, a Nylon 6,6 fiber 12 (Goodfellow company) was immersed vertically into the above mixture and then drawn out of the uncured elastomer pool and cured by rapid heating at ~100°C.

This step could be repeated multiple times to achieve the desired thickness/diameter of the active sheath 14 (Fig. 23). After drying the coated fiber at 30°C for 6 h, the above fiber was then immersed vertically into a freshly mixed PDMS base and curing agent (w/w 10:1, Sylgard 184, Dow Corning) forming the protective sheath 16. The fiber was then drawn out from the uncured PDMS pool and cured by heating at ~90°C. Finally, after drying the above fiber at 30°C for 8 h, the coated precursor fiber 10' was obtained.

### Coiled muscle structure fabrication and characterization

The coiled muscle was obtained by the continuous twisting and coiling process. In a typical experiment, a 200 g weight is hung under one end of the precursor fiber 10', and the other end is fixed to the shaft of a rotating motor. This configuration only allows the fiber 10' to rotate. After the fiber 10' got sufficient turns and can no longer take more twists, the fiber 10' starts to coil on its axis. Next, the coiled muscle structure 10 is stretched with a 32% pre-strain. Then, the fully coiled structure 10 is annealed at above ~180°C for 4 h and evenly cooled down to room temperature to obtain the resulting coiled shape.

Finally, the coiled muscle structure 10 is trained for actuation at 120°C with a 200 g load until consistent actuation is obtained. The SEM measurement was performed on a LEO-1530-VP scanning electron microscope. The RF-magnetic heating system utilized in the current study was EASY HEAT 8310 (Ambrell Induction Heating Solutions). The CCD images and videos were captured by using SONY DSC-RX10III digital camera. The uniform magnetic field was provided by a vibrating sample magnetometer (VSM, EZ7, Microsense).

**Thermal characterization of the protective layer**

The temperature change of the coiled muscle 10 with and without the outmost protective PDMS sheath 16 was recorded under RF-magnetic heating by using a FLIR-A300 camera (FLIR Systems Inc.). As can be seen from Fig. 11, the temperature of the coiled muscle structure 10 without the PDMS sheath 16 rises from 22.7°C to 121°C in air before and after RF-magnetic heating. This is because of the magnetically induced thermal effect of the $Fe_3O_4$ nanoparticles 22. When the coiled muscle structure 10 was coated with a PDMS sheath 16 (Fig. 11), the surface temperature decreased with increasing thickness of the PDMS sheath 16 due to the thermal insulation effect of the PDMS. While there was fracture during the twisting and coiling process, if the PDMS sheath thickness was higher than 200 $\mu$m, the minimum surface temperature of the coiled muscle was 60°C.

**Measurement of the muscle actuation force, torque and actuation strain**

The contractile actuation force of the coiled muscle structure 10 shown in Fig. 13 was measured directly with the Instron tensile measurement machine (INSTRON 5942). As shown in Fig. 24a, the coiled muscle structure 10 was pre-stretched by ~0.1%, and its two ends were directly clamped on the Instron machine's bottom and top gripper. These two grippers were fixed without any further displacement. The tensile actuation force caused by the muscle contraction could thus be obtained. The torsional torque shown in Fig. 2c, 2f was tested with a similar process.

As shown in Fig. 24b, one side of the muscle structure 10 was fixed to the substrate. The other side was connected with the printed bar and tethered on the Instron's gripper. Since it is difficult to use the Instron machine and RF-magnetic heating system at the same time, a heat gun (HE 20-600, Metabo company) was placed to heat the coiled muscle structure 10, and a thermal camera was used to monitor the temperature. The actuation strain of the coiled muscle structure 10 under various loads when heated by heat gun and RF-magnetic heating, respectively, has been compared. As shown in Fig. 25, there is a negligible difference in the actuation strain between these two methods. It indicates that a heat gun is also useful for measuring the performance of a coiled muscle structure 10. The results of the actuation strain shown in Figs. 2a and 2d were characterized under RF-magnetic heating by applying nonrotating loads on the bottom end (Fig. 24c), which provided the tensile force here. Each point in Figs. 2a, d represents the maximum actuation strain of the coiled muscle 10 during the contraction process under the corresponding loading force.

**Calculation of the work capacity**

The work capacity shown in Fig. 2b, e was defined as the product of actuation strain and tensile force from Fig. 2a, d, respectively, i.e.,

$$Work\ capacity = \frac{l \times actuation\ strain \times tensile\ force}{m} .$$

where, $l$ and m are the length and mass of the coiled muscle 10, respectively. The actuation strain is negatively correlated to the actuation stress. Consequently, the work capacity exhibits peaks (called the 'load-optimized work capacity') in Fig. 2b, e.

**Design of the bistable clamper**

The untethered bistable clamper 400 presented here was constructed by combining a bistable linkage structure with the proposed magnetically-heated coiled muscle structure 10 (Fig. 6a). It consisted of a bistable linkage structure with an embedded pre-stretched elastomer 404 and the coiled muscle structure 10. The 3D-printed bistable clamper 400 was composed of two rigid hinged linkages 402. The clamping force of the clamper 400 without connecting the pre-stretched elastomer 404 was measured to be ~1 N. The pre-stretched elastomer 404 that connects two ends of the bistable mechanism enabled the storage and release of the potential strain energy.

**Calculation of the force and work capacity of the magnetic soft actuator**

In this section, we build a simplified model to study how much work capacity a typical magnetic soft actuator could generate. As shown in Fig. 9, it is assumed that the actuator 10 has a residual magnetic flux density aligned with its longitudinal direction, that one end of the beam is fixed while the other end is under a constant external load, and that the beam is applied with a uniform magnetic field along the vertical direction. All these assumptions tend to generate a relatively large magnetic torque and thus result in a relatively large end-effector reaction force. This can help to predict, from the power perspective, the maximum capacity of a typical magnetic soft actuator in mechanical output.

When the magnetic field is not applied, the total potential energy of the actuator with the free end-loaded (the convex shape shown in Fig. 9) is:

$$U_t = \int_V \frac{1}{2} E I \mathrm{K}^2 dV + Fd \quad , \tag{1}$$

where the first term represents the bending energy in the soft actuator, and the second term represents the work done by the external force, *F. EI* is the bending stiffness of the soft bending actuator with E being the Young's modulus of a mag-netic soft composite and $I = \frac{1}{12} w t^3$ being the second moment of inertia. $K \approx \frac{\theta}{L}$ is the approximate curvature in the soft actuator (where $\theta$ is the bending angle of the actuator). $d = L \frac{1 - \cos(\theta)}{\cos(\theta)}$ is the deflection of the beam at the free end. It needs to be noted that, to simplify the model, the self-weight of the actuator is ignored and the linear elasticity for the soft composite is assumed. It is also assumed that the beam is under pure bending and has a uniform curvature, which means that $\theta$ is a constant. The equilibrium bending angle under the constant load of the beam, $\theta_0$, can be numerically solved by minimizing the total potential energy, i.e.,

$$\frac{dU_t}{d\theta} = 0 \quad . \tag{2}$$

With the magnetic field applied, the magnetic moment of the actuator (the concave shape shown in Fig. 9) is:

$$m = \iiint \frac{1}{\mu_0} B_r dV_m \quad , \tag{3}$$

where $B_r$ is the residual flux density, $\mu_0$ is the permeability of the vacuum, and $V_m$ is the volume of the magnetic (NdFeB) microparticles 22. The potential energy induced by the magnetic actuation is defined as:

$$U_m = -\vec{m} \cdot \vec{B} \quad , \tag{4}$$

where *B* is the external magnetic field. By substituting Eq. 3 into Eq. 4, one can get:

$$U_m = -B \frac{B_r}{\mu_0} \cdot wt \int_L \cos(90^o - \varphi) dl \quad , \tag{5}$$

where *w, t,* and L are the width, thickness, and length, respectively. $\varphi$ is the angle between the magnetic moment and the horizontal direction. The work done by magnetic field in rotating the actuator from the loaded state (bending angle $-\theta_0$) to the actuated state (bending angle $\theta$) is:

$$U_m = B \frac{B_r}{\mu_0} \cdot wt \int_0^{\theta_0} \sin(\varphi) \frac{L}{\theta_0} d\varphi - B \frac{B_r}{\mu_0} \cdot wt \int_0^{\theta} \sin(\varphi) \frac{L}{\theta} d\varphi \quad . \tag{6}$$

Then the total potential energy of the actuator is:

$$U_t = B\frac{B_r}{\mu_0} \cdot wt\int_0^{\theta_0}\sin(\varphi)\frac{L}{\theta}d\varphi - B\frac{B_r}{\mu_0} \cdot wt\int_0^{\theta}\sin(\varphi)\frac{L}{\theta_0}d\varphi + \int_V \frac{1}{2}EI(K^2 - K_0^2)dV + F\left[d-(-d_0)\right]$$ , (7)

where $K_0$ and $d_0$ are the curvature and deflection of the soft actuator at loaded state, respectively. $K$ and $d$ are the curvature and deflection of the soft actuator at actuated state, respectively. The equilibrium bending angle upon actuation of the beam can be numerically solved by $\frac{dU_t}{d\theta} = 0$. With the obtained $\theta_{eq}$, one can calculate the work capacity of the actuator by:

$$w = \frac{F\left[d-(-d_0)\right]}{L \cdot w \cdot t}$$ , (8)

With this equation, one can predict the work capacity of the actuator as a function of the magnetic field as shown in Fig. 10.

In Fig. 10, F= 60 $\mu$N, $E$ = 200 kPa, L = 3 mm, $w$ = 1 mm, $t$ = 0.1 mm, $B_r$ = 0.84 T, and $V_m/V$ =0.3 (NdFeB particles/Ecoflex in volume ratio) is used. The result shows that the work capacity increases monotonically first with the magnetic field and then approaches a plateau at $B$ = ~7.8 mT, exhibiting a highly nonlinear behavior. Further increasing the magnetic field beyond $B$ = ~7.8 mT does not significantly change the work capacity, which remains constant at ~2×10$^{-4}$ kJ/kg. This plateau stage is due to the full alignment of the magnetic moment with the magnetic field. It needs to be noted that $E$ = 200 kPa is chosen because the Young's modulus (E) of the magnetic soft actuator is within the range of 6.6-200 kPa. Furthermore, $F$ = 60 $\mu$N is used because an external load larger than 60 $\mu$N will lead to a huge deflection, or even collapse, of the soft beam at the loaded state (according to the numerical result by Eq. 2), thus making the actuator not able to function as designed and be useful. Note that the output force is obtained from solving the relation between the output force and the deflection of the magnetic soft actuator (Eq. 2) by using a numerical method (the corresponding Matlab code is shown in Fig. 26).

It is observed that although assumptions made for the model (a large $B_r$, a homogeneous magnetic moment, a perpendicular magnetic field to the residual magnetic flux density, and a saturated NdFeB-ecoflex volume ratio) tend to generate a large torque (i.e. high force) in the magnetic soft actuator, its maximum work capacity is still only ~2×10$^{-4}$ kJ/kg, which is ~10$^2$ weaker than skeletal muscle, and ~10$^4$ weaker than the proposed magnetic artificial muscle. Unfortunately, in reality, a magnetic soft actuator normally has a heterogeneous magnetic moment, a smaller angle between the magnetic moment and magnetic field, less volume ratio of magnetic particles, and different boundary conditions (e.g. two ends are free), all of which tend to generate a smaller torque. Therefore, it is expected that their "real-life" work capacity to be much smaller than the result shown in Fig. 10. This explains why a smaller work capacity for the magnetic soft actuator is shown in Table 1.

**Calculation of tensile actuation strain, work capacity and torsional torque**

In this section, a model to calculate the tensile actuation strain, work capacity and torsional torque of the coiled muscle structure is built. For the trimorph muscle structure 10, the Young's modulus of the coiled muscle core 12 ($E_{core}$), active sheath 14 ($E_{active}$) and protective sheath 16 ($E_{protective}$) are measured after the annealing process, which are about 1.2 GPa, 1.04 GPa and 1.5 MPa, respectively, i.e.,

$$E_{core} \approx E_{active}$$

$$E_{core} \gg E_{protective}$$

$$E_{active} \gg E_{protective}$$ . (9)

Therefore, to simplify the model, this three-layer coiled muscle structure 10 is assumed as one-layer helical spring, and $E_{core}$ =1.2 GPa is chosen as the Young's modulus. First, the dependence of the actuation strain ($\varepsilon$) and work

capacity ($W$) on the loading force (F) can be calculated from Fig. 27. There are four states (I-IV) of the coiled muscle structure. State I and II represent the non-actuated states, and III and IV are the actuated states. While State I and III are free states, which means that no load is hung on the bottom end, correspondingly, State III and IV are the loaded states. From Fig. 27, the actuation strain and the work capacity with loading force can be obtained:

$$\varepsilon = \frac{\Delta y}{l} = \frac{y_0 + y_1 - y_2}{l} \quad , \tag{10}$$

$$W = \frac{F \Delta y}{m} = \frac{F(y_0 + y_1 - y_2)}{m} \quad , \tag{11}$$

where $m$, $l$, $y_0$, and $\Delta y$ are the mass, length, free actuation length, and load actuation length of the coiled muscle structure, respectively. According to Hooke's Law, $y_1 = \frac{F}{k_1}$ , $y_2 = \frac{F}{k_2}$ . Therefore, Eq. 10 and Eq. 11 can be rewritten as:

$$\varepsilon = \frac{y_0}{l} + \frac{F}{l}\left(\frac{1}{k_1} - \frac{1}{k_2}\right) \quad , \tag{12}$$

$$W = \frac{F y_0}{m} + \frac{F^2}{m}\left(\frac{1}{k_1} - \frac{1}{k_2}\right) \quad , \tag{13}$$

where $k_1$ and $k_2$ represent the spring stiffness of the non-actuated and actuated coiled muscle structure, respectively.

Therefore, the actuation strain and the work capacity are determined by the free actuation strain $\left(\frac{y_0}{l}\right)$ and the change of inverse spring stiffness $\frac{1}{k_1} - \frac{1}{k_2}$ . To finish the calculation, one has to derive the free ac-tuation strain and the spring stiffness. The free actuation strain $\left(\frac{y_0}{l}\right)$ , according to the coiled-driven actuation mechanism, can be obtained from:

$$\frac{y_0}{l} = \frac{l \Delta T}{N} \quad , \tag{14}$$

where $N$ is the number of coils of the coiled muscle. $\Delta T$ is the torsional stroke, which can be calculated by the following equation:

$$\Delta T = \frac{n}{l} - \frac{n_0}{l_0} \quad , \tag{15}$$

where n is the number of fiber turns for making the helix coiled muscle structure; $n_0$ and $l_0$ are the initial values. According to state of the art literature, the changing fiber turns of the coiled muscle structure are caused by the fiber volume expansion in the actuation process. The relationship is:

$$\frac{n}{n_0} = \left(\frac{V_0}{V}\right)^{\frac{1}{2}} \left(\frac{h}{h_0} \cdot \frac{l^2 - h^2}{l_0^2 - h_0^2}\right) \qquad (16)$$

where $V$ and $h$ are the volume and the length of the constructed fiber, respectively. The zero subscripts represent the corresponding initial values. It is assumed that the change of the fiber length in the tensile actuation process is negligible. Eq. 16 can be simplified to:

$$\frac{n}{n_0} \approx \left(\frac{V_0}{V}\right)^{\frac{1}{2}} = \frac{d_0}{d} \qquad (17)$$

where $d$ is the diameter of the constructed fiber. Therefore, by substituting Eq. 15 and Eq. 17 to Eq. 14, the free actuation strain $\dfrac{y_0}{l}$ can be obtained as:

$$\frac{y_0}{l} = \frac{n_0}{N} \left(\frac{d_0}{d} - 1\right) \qquad (18)$$

In addition, the spring stiffness of the coiled muscle is calculated by using Castigliano's theorem:

$$k = \frac{d^4 G}{8 D^3 N} \qquad (19)$$

where D is the diameter of the coil structure, which is assumed to have a negligible change in the actuation process. G is the shear modulus, i.e.,

$$G = \frac{E}{2(1 + \delta)} \qquad (20)$$

where $\delta$ is the poisson's ratio of the fiber. By combing Eq. 12, 13 and Eq. 18, 19, 20 one can calculate the actuation strain and work capacity:

$$\varepsilon = \frac{n_0}{N} \left(\frac{d_0}{d} - 1\right) + \frac{16FN}{El} (1 + \delta) \left(\frac{D^3}{d_0^4} - \frac{D^3}{d^4}\right) \qquad (21)$$

$$W = \frac{l n_0 F}{mN} \left(\frac{d_0}{d} - 1\right) + \frac{16 F^2 N}{mE} (1 + \delta) \left(\frac{D^3}{d_0^4} - \frac{D^3}{d^4}\right) \qquad (22)$$

For the investigated coiled muscle structure, the corresponding parameters are listed below: the number of coils ($N$) is $N$ = 80; the initial number of fiber turns ($n_0$) is $n_0$ = 278; the diameter of the constructed fiber (d) is $d$ = 0.73 mm and the initial value ($do$) is $d_0$ = 0.65 mm; the length of the coiled muscle ($l$) is $l$ = 11 cm; the diameter of the coil structure (D) is D = 1 mm. Here, to simplify the model, a Young's modulus (E) and poisson's ratio ($\delta$) from the nylon fiber core are chosen, i.e. $E$ = 1.2 GPa, $\delta$ = 0.39 as parameters of the constituent fiber in the model. Therefore, one can predict the dependence of the actuation strain and the work capacity on the loading force by using Eq. 21, 22 and the above parameters. As shown in Fig. 28, the theoretical results and the experiment results could be compared to prove the correctness of the above model.

The mismatch between the theoretical and experimental results can be explained as follows: First, it is assumed that the trimorph structure 10 is a one-layer structure of the coiled structure and the nylon fiber's Young's modulus and poisson's ratio as the parameters of the whole constituted fiber 12 are utilized. Next, in this model, the change of the fiber's Young's modulus between the actuated and non-actuated states was not considered. Furthermore, just the diameter expansion of the constituted fiber was considered and it was assumed that the fiber length and the diameter coiled structure have negligible change. Finally, in the actuation process, intercoil contact exhibits if the loading force is small, which is not the case in this model.

In addition, according to state of the art literature, the torsional torque (r) can be calculated by the following equation:

$$\tau = \Delta n \frac{JG}{l}$$

$$(23)$$

where $J = \pi d^4/32$ is the polar second moment of area; $\Delta n = n - n_0$ is the changing number of fiber turns due to the fiber volume expansion in the actuation process. Combing Eq. 17, 20 and 23, the torsional torque can be obtained as:

$$\varepsilon = \frac{\pi d^4 G n_0}{32l}\left(\frac{d_0}{d} - 1\right) = \frac{\pi d^4 E n_0}{64l(1+\delta)}\left(\frac{d_0}{d} - 1\right).$$

$$(24)$$

This equation indicates that the torsional torque increases with an increasing material's Young's modulus and fiber diameter, following the same trend as in the experimental results of Figs. 2c, f.

## Modeling of the bistable clamper

As schematically illustrated in Fig. 6b, the working principle of the bistable clamper 400 follows four steps. First, the elastomer 404 is pre-stretched and attached to both ends of the linkages 402 to enable bistability. The linkage-based structure with embedded pre-stretched elastomer 404 results in an unstable state (state II in Fig. 6b), which has the maximum potential energy as shown in the schematic energy profile of Fig. 6c (state II) and a zero joint revolute angle $\theta$. Second, after releasing the elastomer 404, the clamper rotates and deforms into a concave shape (Fig. 6b) and rests in one stable state (state I in Fig. 6c), which has local minimum energy at $\theta = -\theta_s$ (here the angular position limit of the bistable mechanism is set to stop its rotational movement at preset stopping angles, $\theta_s$, as shown in Fig. 6c). Then a stress-free coiled muscle structure 10 is attached to connect the two arms of the clamper 400, as shown in Fig. 6b. Third, with the contraction of the coiled muscle structure 10 upon magnetic heating, the clamper 400 deforms toward the unstable state, where the elastomer 404 stores the most energy. Fourth, when the device bypasses the unstable state II, it rapidly snaps to the other stable state III with $\theta = \theta_c$ ($\theta_c$ is the revolting angle at which the target tissue is clamped, Fig. 6c). By setting $\theta_c \gg \theta_s$, this stable state III has much lower potential energy than the other stable state I.

Fig. 6c schematically shows the energy landscape of the bistable actuator as a function of the bending angle $\theta$. It shows one peak energy state (unstable state II) and two localized lower energy states (stable states I and III). The difference between the peak and the lower energy state defines the energy barrier. Here the energy barrier from state I to state II is defined as $\Delta E_1$, and the released energy from state II to state III as $\Delta E_2$. To enable the actuation from state I to state II, the muscle actuation should provide sufficient energy input to trigger the snap-through instability, i.e. $U_{in} \geq \Delta E_1$. Once bypassing state II, the bistable system will rapidly snap to the other stable state III, during which a huge amount of stored strain energy in elastomer will be quickly released, resulting in a large energy output $\Delta E_2$. It is observed that by setting $\theta_c \gg \theta_s$, the energy output of this system will be much larger than the input, i.e. $\Delta E_2 \gg U_{in} \geq \Delta E_1$, showing an energy/force amplifying effect.

**Energy-based theoretical model and evaluation of the clamping force**

An energy-based theoretical model is developed to understand the nonlinear behavior of the proposed bistable clamper 400, including the relationship among design parameters (e.g. linkage stop angle and pre-stretched strain in elastomer) and outputs (e.g. potential energy and output force), as shown below. The total potential energy of the bistable clamper is:

$$U_t = U_{muscle} + U_{elastomer} \qquad (25)$$

where $U_{muscle}$ is the potential energy in the coiled artificial muscle structure 10 and $U_{elastomer}$ is the potential energy in the pre-strained rubber 404. In this case, the rubber has a high modulus (~2 MPa) and is pre-stretched at a high strain. Thus, its potential energy will be much larger than that of the twisting muscle structure, i.e. $U_{elastomer} \gg U_{muscle}$. In this case, the second term in Eq. 25 can be ignored. Then Eq. 25 can be rewritten as:

$$U_t = U_{elastomer} = \frac{1}{2} \int E\varepsilon^2 dV \qquad (26)$$

where $E$ is Young's modulus of the elastomer 404 and V is the volume of the elastomer 404. $\varepsilon$ is the strain of the elastomer at a given joint rotation angle $\theta$ as:

$$\varepsilon = \cos(\frac{\theta}{2}) \cdot (1 + \varepsilon_{pre}) - 1 \qquad (27)$$

where $\varepsilon_{pre}$ is the pre-stretched strain of the elastomer 404 at unstable state II. Then one can rewrite Eq. 26 as:

$$U_t = \frac{1}{2} \int E\left[ \cos(\frac{\theta}{2}) \cdot (1 + \varepsilon_{pre}) - 1)^2 \right] dV. \qquad (28)$$

It should be noted that an idealized linear elastic behavior in the homogenized continuous material is assumed despite the nonlinear deformation in the elastomer. Based on the total potential energy of the system, $U_t$, in Eq. 28, the joint torque, T, of the bistable actuator 400 can be obtained by:

$$T = \frac{dU_t}{d\theta}. \qquad (29)$$

The clamping force of the bistable actuator 400, $F_c$, or the reaction force of the end-effector, can be obtained by:

$$F_c = \frac{T}{d}, \qquad (30)$$

where $d$ is the distance between the clamping tip and the joint, as shown in Fig 6d. Based on this equation, the clamping force can be plotted as a function of joint rotation angle, $\theta$, as shown in Fig. 6d. It shows that by setting $\theta_s$ = 4°, $E$ = 2 MPa and $\varepsilon_{pre}$ = 400%, the force required to bypass the energy barrier is ~1.46 N. Once the onset of the snap-through instability occurs, the structure snaps and the corresponding output force can reach ~18.09 N at $\theta_c$ = 50°, showing a ~10.70 times amplification in output force. To validate the model, the static output force of the bistable clamper is experimentally examined as a function of bending angle through a quasi-static indenting test with the set-up shown in Fig. 29. Similar to the trend captured by the model, the experiment result shows that by increasing the difference between $\theta_s$ and $\theta_c$, e.g., $\theta_s$ = 4° and $\theta_c$ = 50°, the system shows a huge force amplification by a factor of up to -7.43 (the mismatch between the model and experiment is explained in detail below). To better understand the force amplification effect, the corresponding output force/force input is plotted as a function of $\theta_c/\theta_s$ in Fig. 30. It shows that the force-improved-ratio of the bistable system increases monotonically with $\theta_c/\theta_s$, indicating that one can simply boost the output force/input force from 1 to 7.43 by simply tuning $\theta_c/\theta_s$ from 1 to 13. In addition to the joint angle, the energy and force landscape of the bistable system can also be tuned by pre-stretched strain, $\varepsilon_{pre}$, in the elastomer, as shown in Fig. 31, where the clamping force and energy output increased markedly with $\varepsilon_{pre}$.

The analytical model and the experiment results only capture the static (or quasi-static) response of the bistable systems. If one includes the dynamic effects of the system during the snap-through process, its output force would be much larger than the force modeled (and measured) here because a fast snap-through instability normally contributes to a larger dynamic force. The mismatch between the model and the experiment results shown in Fig. 6d is explained as below. First, in the model, the potential energy of the twisting muscle is ignored. In reality, the strain energy in twisting muscles will significantly increase at a large bending angle. This explains why only a small mismatch between model and experiment is observed at small $\theta$, but a large discrepancy is observed at large $\theta$. Next, in the model, it is assumed that the friction at the joint is zero. In reality, the joint friction is not zero, and it increases significantly with $\varepsilon_{pre}$. Third, a linear elasticity for the elastomer in the model is assumed. According to the measured stress-strain curve of the elastomer, as shown in Fig. 32, it shows a highly non-linearity despite a nearly constant slope at a small strain.

## Claims

1. A device composed of one or more structures (10), each of said structures (10) comprising at least three layers:

    a core (12),
    an active sheath (14); and
    a protective sheath (16),
    wherein the core (12) is formed by nylon fibers (18),
    wherein the active sheath (14) comprises a resin (20), with graphene oxide (GO) and a first type of magnetic particles (22) being respectively embedded in the resin (20); and
    wherein the protective sheath (16) is formed from an elastomeric material (24) that protects the inner layers from an external environment.

2. The device according to claim 1,
    further comprising a fourth layer (24) with the fourth layer being a magnetization sheath (24).

3. The device according to claim 2,
    wherein the magnetization sheath (24) comprises a combination of a second type of magnetic particles (28) together with an elastomer (30), such as a combination of NdFeB with PDMS.

4. The device according to one of the preceding claims,
    wherein the resin (20) of the active sheath (14) comprises a urethane casting resin, such as Crystal Clear™, or nylon.

5. The device according to one of the preceding claims,

    wherein the first type of magnetic particles (22) is formed by $Fe_3O_4$, with the first type of magnetic particles (22) comprising a size selected in the range of 10 to 500 nm, especially in the range of 20 to 200 nm, in particular in the range of 50 to 100 nm,
    and/or with the device further comprising the second type of magnetic particles (28) being formed by NdFeB, with said second type of magnetic particles (28) comprising a size selected in the range of 0,1 to 10 $\mu$m, especially in range of 2 to 6 $\mu$m.

6. The device according to one of the preceding claims,
    wherein the active sheath (14) comprises a thickness in the range of 50 to 300 $\mu$m, in particular of 100 to 250 $\mu$m, especially 200 $\mu$m.

7. The device according to one of the preceding claims,
    wherein the active sheath (14) comprises a GO concentration selected in the range of 1 to 6 wt%, especially in the range of 2 to 4 wt%.

8. The device according to one of the preceding claims,
    wherein the active sheath (14) comprises a magnetic particle concentration of the first type (22) of magnetic particles selected in the range of 10 to 30 wt%.

9.  The device according to one of the preceding claims,
    wherein the device comprises a coiled shape.

10. The device according to one of the preceding claims,
    wherein the core (12), i.e. the nylon fibers (12), comprise a diameter selected in the range of 0.1 to 0.5 mm, especially of 0.2 to 0.5 mm, in particular of 0.45 mm, and preferably a length selected in the range of 1 to 100 cm, in particular in the range of 1 to 50 cm, especially in the range of 5 to 40 cm.

11. The device according to one of the preceding claims,
    wherein the device comprises a diameter selected in the range of 0.1 to 10 mm, in particular of 0.4 to 5 mm, especially of 0.4 to 1.2 mm.

12. The device according to one of the preceding claims,
    wherein the device comprises at least one functional component such as a frame (202), blades (206), bars (502), a screw, a chassis (402, 102), a connector (504), a magnetic part (204), a pretensioned elastomer (404) and/or combinations of the foregoing.

13. A method of forming a device having one or more three layered structures (10), the method comprising the steps of:

    providing a fiber (12);
    coating the fiber (12) with an active sheath (14) material, wherein the active sheath material comprises a resin (20), with graphene oxide (GO) and a first type of magnetic particles (22) being respectively embedded in the resin;
    further coating the fiber (12) with a protective sheath (16) material, wherein the protective sheath (16) material comprises elastomeric material (24);
    optionally curing said coated fiber (10); and
    continuously twisting and coiling said coated fiber (10) to form the device.

14. The method according to claim 14,
    wherein the method further comprises the step of coating the fiber with an additional magnetic sheath (26) before coiling the coated fiber (10).

15. The method according to one of claims 13 or 14,
    wherein the step of coiling said coated fibers (10) comprises at least 250 turns, preferably 300 to 400 turns.

Fig.1

Fig.2

Fig.3

Fig.4

Fig.5

*Fig.6*

*Fig.7*

| Type | Actuation strain (%) | Work capacity (kJ/kg) | Energy density $(kJ/m^3)$ | Ref. |
|---|---|---|---|---|
| Coiled artificial muscle | ~30.6 | ~3.5 | ~2000 | this work |
| Skeletal muscle | ~30 | ~0.07 | ~20 | 28 |
| McKibben actuator | ~38 | ~1.1 | ~0.28 | 29 |
| LCE | ~30 | ~0.03 | ~30 | 30 |
| Bio-hybrid | ~15 | ~0.001 | ~1 | 31 |
| HASEL | ~60 | ~0.07 | ~0.23 | 32 |
| IPMC | ~3 | ~0.001 | ~3 | 33 |
| Magnetic soft robot | ~50 | $~2 \times 10^{-4}$ | ~0.15 | 'Calculation of the force and work capacity of the magnetic soft actuator' section |

*Fig.8*

Fig.9

Fig.10

*Fig.11*

EP 4 233 765 A1

a) Nylon fiber

10

12

Crystal clear
+
GO
+
Fe$_3$O$_4$ NP

14

PDMS

16

0.1 mm

b)

10

0.5 mm

*Fig.12*

*Fig.13*

*Fig.14*

*Fig.15*

: Strain distribution

*Fig.16*

*Fig.17*

*Fig.18*

Fig.19

400

404

402

muscle contraction

SMM elogation

404

402

*Fig.20*

Active sheath

Protective sheath

12

10

14

Muscle
core

16

26

Magnetization sheath

*Fig.21*

*Fig.22*

*Fig.23*

a)

b)

c)

10

10

10

10

━━━ : Substrate    ⊏══⊐ : Coiled muscle    ⊏══⊐ : Printed bar

▨▨▨ : Nonrotating loads    ⊏══⊐ : Instron component

*Fig.24*

*Fig.25*

```
clear;clc;close all;
L=3*10^-3;    %the length of beam (m)
w=1*10^-3;    % the widht of beam (m)
t=.1*10^-3;   % the thickness of beam (m)
Br=0.84;      % the residual flux density (T)
mu0=4*pi*10^-7;  %permeability of the vacuum (H/m)
E=200*1000;   %Modulus of the beam (Pa)
V=L*w*t;      %volume
F=6e-5;       % applied force (N)


n=100;
thetap=linspace(0,pi,n);
Ut=(1/24)*E*w/0.3*t^3*thetap.^2/L-F*L*(1-cos(thetap))./thetap;
for i=1 :size(Ut,2)
    x=find(Ut==min(Ut));          %numerically solve dUt/dtehta=0
end
ctheta=thetap(x);
d0=L*(1-cos(ctheta))/ctheta
```

Command Window

New to MATLAB? See resources for Getting Started.

```
d0 =

    0.0020
```

*Fig.26*

*Fig.27*

*Fig.28*

Compression

400

: Substrate    : Instron component

: Fixed auxiliary structure

*Fig.29*

$\dfrac{\text{Force output}}{\text{Force intput}}$

— model
● exp

$|\theta c/\theta s|$

*Fig.30*

*Fig.31*

*Fig.32*

Europäisches
Patentamt

European
Patent Office

Office européen
des brevets

# EUROPEAN SEARCH REPORT

**Application Number**

EP 22 15 8571

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 082 248 A2 (UNIV TEXAS [US]) 19 October 2016 (2016-10-19) * clauses 50, 64, 67, 102; paragraphs [0013], [0054] - [0055], [0085], [0091] - [0092], [0100], [0115], [0203] * | 1-15 | INV. A61B34/30 F03G7/06 |
| Y | BASTOLA ANIL K ET AL: "The shape - morphing performance of magnetoactive soft materials", MATERIALS & DESIGN, ELSEVIER, AMSTERDAM, NL, vol. 211, 13 October 2021 (2021-10-13), XP086852396, ISSN: 0264-1275, DOI: 10.1016/J.MATDES.2021.110172 [retrieved on 2021-10-13] * 1. Introduction; 2.1.1 Matrices; 2.1.2 Magnetic fillers; 4.4 Multi-stimuli actuation (magnetic field + other stimuli); 5. Applications of MSMs * | 1-15 | |
| A | WO 2015/157621 A1 (HARVARD COLLEGE [US]) 15 October 2015 (2015-10-15) * page 9, lines 17-24 * | 1-15 | TECHNICAL FIELDS SEARCHED (IPC)<br><br>A61B<br>F03G |
| A | WO 2021/183924 A1 (MASSACHUSETTS INST TECHNOLOGY [US]) 16 September 2021 (2021-09-16) * the whole document * | 1-15 | |
| A | WO 2020/231741 A2 (UNIV TEXAS [US]) 19 November 2020 (2020-11-19) * the whole document * | 1-15 | |
| A | US 10 473 093 B2 (DAEGU GYEONGBUK INST SCIENCE & TECH [KR]) 12 November 2019 (2019-11-12) * the whole document * | 1-15 | |

The present search report has been drawn up for all claims

| Place of search | Date of completion of the search | Examiner |
|---|---|---|
| The Hague | 19 July 2022 | Rosander, Frida |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 8571

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2022

| Patent document cited in search report | | Publication date | Patent family member(s) | | Publication date |
|---|---|---|---|---|---|
| EP 3082248 | A2 | 19-10-2016 | CN | 104769834 A | 08-07-2015 |
| | | | CN | 105003405 A | 28-10-2015 |
| | | | EP | 2880755 A2 | 10-06-2015 |
| | | | EP | 3082248 A2 | 19-10-2016 |
| | | | HK | 1210549 A1 | 22-04-2016 |
| | | | JP | 6228605 B2 | 08-11-2017 |
| | | | JP | 6343267 B2 | 13-06-2018 |
| | | | JP | 6438557 B2 | 12-12-2018 |
| | | | JP | 6764459 B2 | 30-09-2020 |
| | | | JP | 2015533521 A | 26-11-2015 |
| | | | JP | 2016042783 A | 31-03-2016 |
| | | | JP | 2018068106 A | 26-04-2018 |
| | | | JP | 2019080488 A | 23-05-2019 |
| | | | KR | 20150038475 A | 08-04-2015 |
| | | | US | 2015152852 A1 | 04-06-2015 |
| | | | US | 2015219078 A1 | 06-08-2015 |
| | | | US | 2018073490 A1 | 15-03-2018 |
| | | | US | 2020088175 A1 | 19-03-2020 |
| | | | US | 2020191127 A1 | 18-06-2020 |
| | | | US | 2022003221 A1 | 06-01-2022 |
| | | | WO | 2014022667 A2 | 06-02-2014 |
| WO 2015157621 | A1 | 15-10-2015 | NONE | | |
| WO 2021183924 | A1 | 16-09-2021 | NONE | | |
| WO 2020231741 | A2 | 19-11-2020 | NONE | | |
| US 10473093 | B2 | 12-11-2019 | US | 2018058429 A1 | 01-03-2018 |
| | | | US | 2020040876 A1 | 06-02-2020 |

EPO FORM P0459

For more details about this annex : see Official Journal of the European Patent Office, No. 12/82